# EUROPEAN PATENT APPLICATION

(11) **EP 4 654 222 A1**
(43) Date of publication of application: **26.11.2025**
(21) Application number: 25175943.7
(22) Date of filing: 13.05.2025
(51) Int. Cl.: H01B 1/24

(54) **DEEP EUTECTIC LIQUID, BIO-ELECTRODE COMPOSITION, BIO-ELECTRODE, AND METHOD FOR PRODUCING BIO-ELECTRODE**

(30) Priority: 20.05.2024 JP 2024082068
(71) Applicant: SHIN-ETSU CHEMICAL CO., LTD., Tokyo 1000005 (JP)
(72) Inventor: Ikeda, Joe, Tokyo, 100-0005 (JP); Nonaka, Shiori, Niigata (JP); Hatakeyama, Jun, Niigata (JP)
(74) Representative: Schicker, Silvia

(57) **Abstract**

The present invention is a deep eutectic liquid, which is a mixture of a hydrogen bond donor compound and a hydrogen bond acceptor compound, wherein the hydrogen bond donor compound is a compound represented by the following general formula (1) having a structure in which 2 to 100 monomers having a hydroxy group are bonded, the hydrogen bond acceptor compound is a compound containing a monomer having a quaternary ammonium cation represented by the following general formulae (2) to (6) or a quaternary phosphonium cation represented by the following general formula (7), and the deep eutectic liquid is present in a liquid state at 25°C. The present invention provides a deep eutectic liquid that has high ionic conductivity and ensures safety upon contact with living bodies, a bio-electrode composition containing the deep eutectic liquid capable of promptly acquiring signals by being applied to the skin without leaving residues on the skin, stably obtaining biological signals for a long period, and forming a living body contact layer for bio-electrodes, a bio-electrode in which a living body contact layer is formed from the bio-electrode composition, as well as a method for producing the bio-electrode.

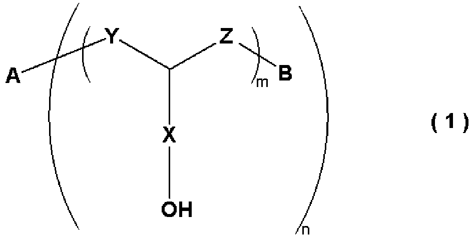

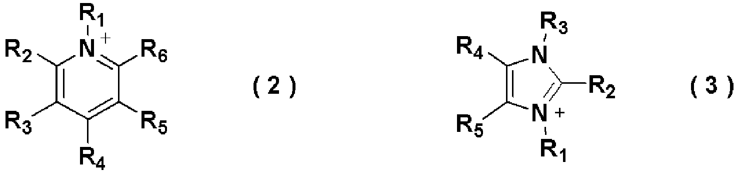

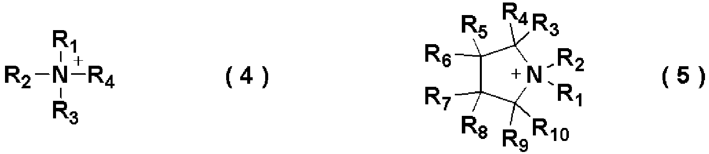

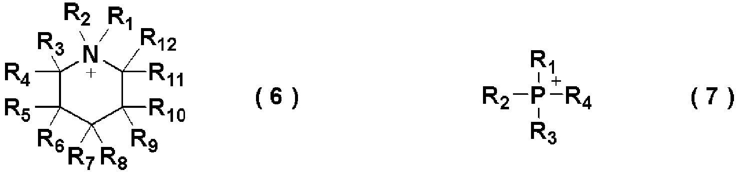

## Description

### TECHNICAL FIELD

The present invention relates to a deep eutectic liquid, a bio-electrode composition, a bio-electrode, and a method for producing a bio-electrode.

### BACKGROUND ART

A recent growing popularity of Internet of Things (IoT) has accelerated the development of wearable devices, such as watches and eye-glasses that allow for Internet access. Even in the fields of medicine and sports, wearable devices for constantly monitoring the user's physical state are demanded, and such technological development is expected to be further encouraged.

In the field of medicine, the use of wearable devices has been examined for monitoring the state of human organs by sensing extremely weak current, such as an electrocardiogram which detects an electric signal to measure the motion of the heart. The electrocardiogram measurement is conducted by attaching electrodes coated with an electro-conductive paste to the body, but this is a short-time measurement and is conducted only once. On the other hand, development of the above medical wearable device is aimed at production of devices for constantly monitoring the health condition for several weeks. Accordingly, a bio-electrode used in a medical wearable device is required to make no changes in electric conductivity and cause no skin allergy even in long-time use. In addition to these, it is also required that a bio-electrode is light-weight and can be produced at low cost.

Medical wearable devices are classified into two types: a type which is directly attached to the body and a type which is incorporated into clothes. As the type to be attached to the body, there has been proposed a bio-electrode using water-soluble gel containing water and electrolytes, which are materials of the above electro-conductive paste (Patent Document 1). The water-soluble gel contains sodium, potassium, or calcium as the electrolytes in a water-soluble polymer for retaining water, and converts changes in ion concentration from the skin into electricity. On the other hand, as the type which is incorporated into clothes, there has been proposed a method using electrodes formed of a cloth in which an electro-conductive polymer such as PEDOT-PSS (poly-3,4-ethylenedioxythiophene-polystyrenesulfonate) or silver paste is incorporated into the fibers (Patent Document 2).

However, the use of the water-soluble gel described above containing water and electrolytes brings about loss of electric conductivity due to water evaporation by dryness. Meanwhile, the use of a higher-ionization-tendency metal such as copper can cause some users to suffer from skin allergy. The use of an electro-conductive polymer such as PEDOT-PSS can also cause skin allergy due to the strong acidity of the electro-conductive polymer, and further cause the electro-conductive polymer to peel off from the fibers during washing.

Further, by taking advantage of excellent electric conductivity, the use of metal nanowire, carbon black, carbon nanotube, and the like as electrode materials has been examined (Patent Documents 3, 4, and 5). With higher contact probability among wires, the metal nanowires can conduct electricity even when added in small quantities. The metal nanowire, however, can cause skin allergies since they are thin materials with sharp tips. Carbon nanotubes are also irritating to the body for the same reason. Carbon black is not as toxic as carbon nanotubes, but is slightly irritating to the skin. Even if these electrode materials themselves cause no allergic reaction in the manners described above, the biocompatibility may be degraded depending on the shape of a material and its skin irritancy, thereby making it difficult to satisfy both electric conductivity and biocompatibility.

Although metal films seem to function as excellent bio-electrodes owing to its extremely high electric conductivity, this is not always the case. Upon heartbeat, the human skin releases not only extremely weak current, but also sodium ion, potassium ion, and calcium ion. It is thus necessary to convert changes in ion concentration into current. However, noble metals are difficult to ionize and are inefficient in converting ions from skin into current. Therefore, in the bio-electrodes containing a noble metal, impedance is high, and resistance is also high during electrical conduction to the skin.

A lithium-ion conductive composite for solid electrolytes prepared by using a silicon atom-containing compound with a segment containing lithium ions has been proposed. The composite exhibits high electroconductivity with desirable lithium-ion transport number at battery operating temperatures while also possessing excellent mechanical properties, moldability, and strong adhesiveness to electrodes. Accordingly, the composite enables the production of high-safety batteries with reduced or eliminated risks of ignition or electrolyte leakage (Patent Document 6).

Bio-electrodes containing ionic polymers have been proposed (Patent Documents 7, 8, 9, and 10). A bio-electrode formed by blending a silicone adhesive with an ionic polymer and carbon powder exhibits adhesiveness, allowing stable acquisition of biological signals even when the electrode is attached to the skin for prolonged periods. Since ionic polymers do not permeate the skin, they do not cause skin irritation and exhibit high biocompatibility.

Silicone is inherently an insulator; however, the combination of ionic polymers and carbon powder enhances its ionic conductivity, enabling its use as a bio-electrode. Nevertheless, further improvements in ionic conductivity are still required to improve the performance.

Patent Documents 7, 8, 9, and 10 mentioned above demonstrate effective use of a silicone compound with polyether chains as an additive for enhancing ionic conductivity. Polyether chains have also been used to enhance ionic conductivity in lithium-ion polymer batteries and have proven effective for enhancing ionic conductivity. However, their ionic conductivity remains lower than that in the hydrogel of the water-soluble gel, necessitating further enhancement in ionic conductivity.

It is necessary for bio-electrodes to allow for signal detection immediately after they are applied to the skin. Gel electrodes facilitate smooth ion exchange due to their similar ion concentration to the skin, and the rapid ion mobility within the hydrogel enables immediate signal detection upon application to the skin. In contrast, dry electrodes require a longer period for signal detection after they are attached to the skin. This delay of signal detection is presumably due to the time needed for ions released from the skin to be saturated on the surface of the dry electrode before signal detection.

Patent Documents 7, 8, 9, and 10 mentioned above also disclose compositions serving as bio-electrodes that contain ionic polymers alone, or ionic polymers combined with resins, conductive particles or compounds containing polyglycerin. However, since ionic polymers exist in a solid state under a dry condition, they are presumed to remain solid also in the dry electrodes. While materials called solid electrolytes have also been known, the ionic conductivity of the ionic polymers as disclosed in Patent Documents 7, 8, 9, and 10 mentioned above has not been confirmed. In addition, liquids generally exhibit higher ionic conductivity than solids, suggesting that ionic polymers localized within dry electrodes may be disadvantageous in terms of ionic conductivity. Therefore, the ionic conductivity of these ionic polymers is likely inferior to that in the hydrogel of the water-soluble gel.

Meanwhile, a deep eutectic liquid has been known as a material that is obtained by mixing a hydrogen bond donor compound with a hydrogen bond acceptor compound. Deep eutectic liquids are characterized by, for example, their liquid state at room temperature, low vapor pressure, non-volatility (unlike water), flame retardance, thermal stability, electrochemical stability, electrical conductivity, low cost, environmental compatibility, and low toxicity. Since deep eutectic liquids exist in a liquid state, their incorporation into dry electrodes is expected to improve ionic conductivity and reduce interfacial resistance with living bodies.

However, since deep eutectic liquids can be formulated in various combinations, it is necessary for those used in dry electrodes to be formed of components that do not cause skin irritation or toxicity. In other words, it is necessary to select each of the hydrogen bond donor compound and the hydrogen bond acceptor compound from biocompatible materials.

Patent Document 11 discloses a food product containing a flavoring composition formed from a deep eutectic liquid. Because food products are intended for human consumption, it is necessary for the deep eutectic liquids for this usage to be biocompatible and nontoxic. Examples of such deep eutectic liquids include amino acids, sugars, and compounds naturally produced in the body. These compounds are considered also suitable for application in dry electrodes.

Incorporating such biocompatible deep eutectic liquids into dry electrodes is expected to enhance ionic conductivity in the dry electrode. Additionally, because deep eutectic liquids are in a liquid state, they can reduce interfacial resistance between the dry electrode and the living body. As a result, signals can be obtained immediately after application to the skin, and biological signals can be stably obtained without baseline drift or the like due to movement.

Further, Patent Documents 12, 13, and 14 disclose conductive resins containing deep eutectic liquids, methods for producing the conductive resins, and sensors having the conductive resins. Certain combinations of deep eutectic liquids can be volatilized to form porous structures. The porous membranes thus produced exhibit high pressure sensitivity and can be used as resistance change sensors by being attached to the human body to obtain biological information. However, since the resistance variations contribute to noise in bio-electrodes, the conductive resins produced in this manner are incapable of stable signal acquisition in bio-electrodes.

Further, Patent Documents 15 and 16 disclose bio-electrodes and wearable devices containing ionic liquids. Ionic liquids are liquid materials with properties similar to those of deep eutectic liquids, and are expected to exhibit an effect of reducing interfacial resistance with the living body when they are incorporated into bio-electrodes. However, many ionic liquids contain compounds with biological toxicity, limiting their use in biocompatible materials. In fact, 1-butyl-3-methylimidazolium tetrafluoroborate and 1-ethyl-3-methylimidazolium tetracyanoborate disclosed in Patent Documents 15 and 16 are known to cause chemical burns or transdermal toxicity.
Therefore, their application is likely limited to short-term use for the body, rather than long-term use.

As described above, although it is necessary for bio-electrodes for wearable devices to allow for signal acquisition immediately after the application to the skin and also maintain stable signal acquisition for a long period, the development of dry electrodes has faced numerous challenges. Accordingly, there is a need to develop a bio-electrode that is formed of materials having high ionic conductivity and ensuring safety upon contact with living bodies, while also allowing easy attachment and detachment to the body without causing skin irritation or leaving residues, enabling stable long-term acquisition of biological signals immediately after the application to the skin.

### CITATION LIST

### PATENT LITERATURE

Patent Document 1: WO 2013/039151 A1
Patent Document 2: JP 2015-100673 A
Patent Document 3: JP H05-095924 A
Patent Document 4: JP 2003-225217 A
Patent Document 5: JP 2015-019806 A
Patent Document 6: JP 2007-059092 A
Patent Document 7: JP 2019-180467 A
Patent Document 8: JP 2021-115458 A
Patent Document 9: JP 2022-075537 A
Patent Document 10: JP 2022-075544 A
Patent Document 11: JP 2016-538405 A
Patent Document 12: JP 2022-085568 A
Patent Document 13: JP 2022-085569 A
Patent Document 14: JP 2023-176396 A
Patent Document 15: JP 2015-016166 A
Patent Document 16: JP 2015-077226 A

### SUMMARY OF INVENTION

### TECHNICAL PROBLEM

The present invention has been made to solve the above problems, and an object of the present invention is to provide a deep eutectic liquid having high ionic conductivity and ensuring safety upon contact with living bodies, a bio-electrode composition containing the deep eutectic liquid capable of promptly acquiring signals by being applied to the skin without leaving residues on the skin, stably obtaining biological signals for a long period, and forming a living body contact layer for bio-electrodes, a bio-electrode in which a living body contact layer is formed from the bio-electrode composition, as well as a method for producing the bio-electrode.

### SOLUTION TO PROBLEM

In order to solve the above problems, the present invention provides a deep eutectic liquid, which is a mixture of a hydrogen bond donor compound and a hydrogen bond acceptor compound, wherein the hydrogen bond donor compound is a compound represented by the following general formula (1) having a structure in which 2 to 100 monomers having a hydroxy group are bonded, the hydrogen bond acceptor compound is a compound containing a monomer having a quaternary ammonium cation represented by the following general formulae (2) to (6) or a quaternary phosphonium cation represented by the following general formula (7), and the deep eutectic liquid is present in a liquid state at 25°C, 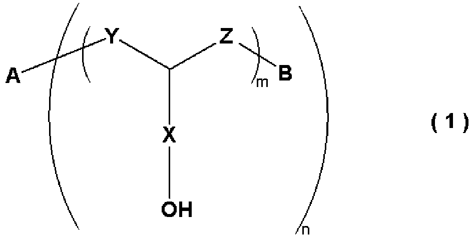 wherein X is a single bond, or a linear, branched, or cyclic divalent hydrocarbon group having 1 to 30 carbon atoms, which may be substituted with a heteroatom or intervened by a heteroatom; Y and Z each represent a linear, branched or cyclic divalent hydrocarbon group having 1 to 5 carbon atoms, which may be substituted with a heteroatom or intervened by a heteroatom; A and B each represent a hydrogen atom, a hydroxy group, an amino group, a halogen atom, or an alkyl group or an alkyl group with a terminal substituted with a siloxane that may be substituted with a heteroatom or intervened by a heteroatom; Y and Z may be identical to or different from each other; A and B may be identical to or different from each other; "m" is an integer of 1 to 100 and represents a repetition of chemical structural units, and "n" is an integer of 1 to 4 and represents a repetition of chemical structural units, provided that 2 ≤ m×n ≤ 100 is satisfied, 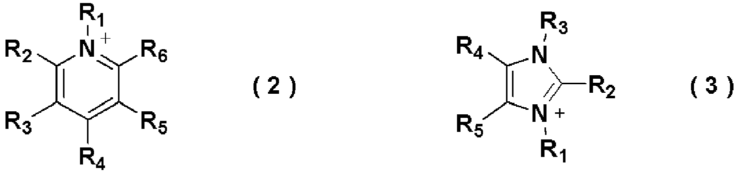 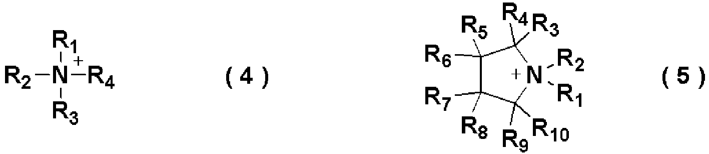 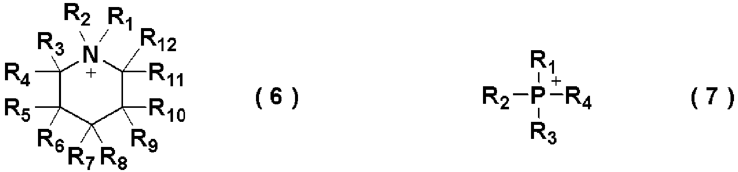 wherein R₁ to R₁₂ each represent a linear, branched, or cyclic monovalent hydrocarbon group having 1 to 30 carbon atoms that may be substituted with a heteroatom or intervened by a heteroatom and may form a zwitterion having an anionic portion, or a hydrogen atom, a hydroxy group, an amino group, a nitro group, or a halogen atom; R₁ to R₁₂ may be identical to or different from one another.

Such a deep eutectic liquid has high ionic conductivity and ensures safety upon contact with living bodies.

Further, in the present invention, each monomer having a hydroxy group is preferably glycerin.

Such a deep eutectic liquid has higher ionic conductivity and ensures superior safety upon contact with living bodies.

Here, the hydrogen bond donor compound is preferably a polyglycerin-modified silicone represented by the following general formula (8) or (9), 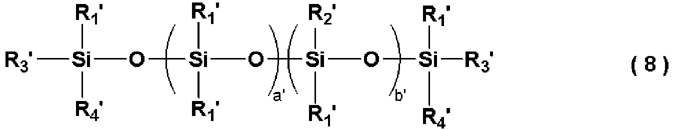 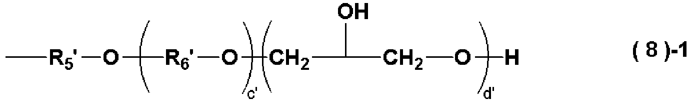 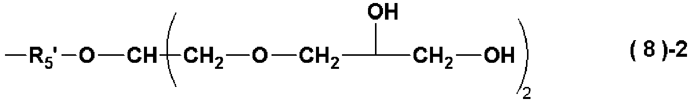 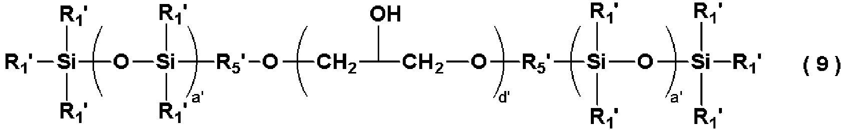 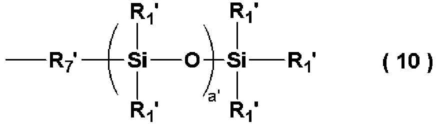 wherein R₁' is identical to or different from one another, and independently represents a hydrogen atom, a phenyl group, a linear or branched alkyl group having 1 to 50 carbon atoms, or a silicone chain represented by the general formula (10), and optionally contains an ether group; R₂' represents a group having a polyglycerin structure represented by the general formula (8)-1 or (8)-2; R₃' is identical to or different from one another, and independently represents the R₁' or the R₂'; R₄' is identical to or different from one another, and independently represents the R₁', the R₂', or an oxygen atom; when R₄' is an oxygen atom, two R₄' moieties may bond to each other to form an ether group and may form a ring together with silicon atoms to which they are bonded; "a'" is identical to or different from one another and represents 0 to 100, "b'" is 0 to 100, and a'+b' is 0 to 200, provided that, when "b'" is 0, at least one of R₃' is the R₂'; R₅' represents an alkylene group having 2 to 10 carbon atoms or an aralkylene group having 7 to 10 carbon atoms; R₆' and R₇' each represent an alkylene group having 2 to 6 carbon atoms; R₇' may be an ether group; "c'" is 0 to 20; and "d'" is 2 to 20.

With the deep eutectic liquid containing the above compound, it is possible to provide a bio-electrode formed using a bio-electrode composition containing the deep eutectic liquid that exhibits both biocompatibility and ionic conductivity, is capable of promptly acquiring signals by being applied to the skin, and stably obtaining biological signals for a long period.

Further, the present invention provides a bio-electrode composition containing the deep eutectic liquid described above.

Such a bio-electrode composition can form a living body contact layer for bio-electrodes, which is capable of promptly acquiring signals by being applied to the skin without leaving residues on the skin, and stably obtaining biological signals for a long period.

Further, in the present invention, the bio-electrode composition preferably contains a binder (A).

By incorporating the binder (A) in the bio-electrode composition, it is possible to prevent elution of the deep eutectic liquid and to impart adhesion.

Here, it is preferable that the binder (A) be one or more resins selected from the group consisting of a silicone resin, a polyurethane resin, and a polyacrylic resin.

Such a binder (A) can provide good adhesion to the living body without leaving residues on the skin.

Further, in the present invention, it is preferable that the bio-electrode composition contain a conductive particle (B).

Here, the conductive particle (B) preferably contains one or more selected from the group consisting of carbon powder, gold, silver, silver chloride, platinum, aluminum, magnesium, tin, tungsten, iron, copper, nickel, stainless steel, chromium, titanium, molybdenum, ruthenium, and indium.

The carbon powder is preferably one or both of carbon black and carbon nanotube.

Such conductive particles (B) can reduce interfacial resistance between the bio-electrode and a living body.

Further, in the present invention, the bio-electrode composition preferably further contains glycerin.

By incorporating glycerin in the bio-electrode composition, the interfacial resistance with the living body can be further reduced, and the ionic conductivity can be further enhanced.

Further, the present invention provides a bio-electrode containing a conductive base material and a living body contact layer formed on the conductive base material, wherein the living body contact layer contains a cured product of the bio-electrode composition described above.

Since the bio-electrode of the present invention contains the living body contact layer that contains a cured product of the bio-electrode composition described above, it exhibits excellent electric conductivity and biocompatibility, is lightweight, and can be produced at low cost. The bio-electrode of the present invention also prevents significant reduction in electric conductivity even when exposed to moisture or drying, and enables prompt signal acquisition upon application to the skin without leaving residue on the skin.

Here, the conductive base material preferably contains one or more selected from the group consisting of gold, silver, silver chloride, platinum, aluminum, magnesium, tin, tungsten, iron, copper, nickel, stainless steel, chromium, titanium, and carbon.

As described above, various conductive base materials can be used for the bio-electrode of the present invention.

Further, the present invention provides a method for producing a bio-electrode having a conductive base material and a living body contact layer formed on the conductive base material, the method includes applying the bio-electrode composition described above onto the conductive base material, and curing the bio-electrode composition to form the living body contact layer.

Such a method for producing a bio-electrode is capable of easily and inexpensively producing a bio-electrode that exhibits excellent electric conductivity and biocompatibility, is lightweight, and prevents significant reduction in electric conductivity even when exposed to moisture or drying, and also enables prompt signal acquisition upon application to the skin.

Here, a conductive base material preferably contains one or more selected from the group consisting of gold, silver, silver chloride, platinum, aluminum, magnesium, tin, tungsten, iron, copper, nickel, stainless steel, chromium, titanium, and carbon.

As described above, various conductive base materials can be used for the method for producing a bio-electrode of the present invention.

Further, the present invention provides another method for producing a bio-electrode having a conductive base material and a living body contact layer formed on the conductive base material, the method includes applying the bio-electrode composition described above onto a release substrate, followed by curing of the bio-electrode composition, and patterning of the cured product, and transferring the patterned product onto the conductive base material to form the living body contact layer.

Such a method for producing a bio-electrode is capable of easily and inexpensively producing a bio-electrode that exhibits excellent electric conductivity and biocompatibility, is lightweight, and prevents significant reduction in electric conductivity even when exposed to moisture or drying, and also enables prompt signal acquisition upon application to the skin. The method can also provide designability of the bio-electrode.

### ADVANTAGEOUS EFFECTS OF INVENTION

As described above, the deep eutectic liquid according to the embodiment of the present invention has biocompatibility and ionic conductivity and can be used for a bio-electrode composition. Further, the bio-electrode composition according to the embodiment of the present invention is capable of forming a living body contact layer for a bio-electrode, which exhibits excellent electric conductivity and biocompatibility, is lightweight, can be produced at low cost, prevents significant reduction in electric conductivity even when exposed to moisture or drying, enables prompt signal acquisition upon application to the skin without leaving residues on the skin, and also enables stable long-term measurement of biological signals.

### BRIEF DESCRIPTION OF DRAWINGS

FIG. 1 is a schematic cross-sectional view showing an example of a bio-electrode of the present invention;
FIG. 2 is a schematic cross-sectional view showing an example of a bio-electrode of the present invention worn on a living body;
FIG. 3 is a schematic-view of printed bio-electrodes prepared in Examples of the present invention;
FIG. 4 is a schematic-view of one of the bio-electrodes prepared in Examples of the present invention, which is cut out and provided with an adhesive layer;
FIG. 5 is a view showing locations where electrodes and earth are attached onto a human body in measuring biological signals in Examples of the present invention; and
FIG. 6 shows one of electrocardiogram waveforms obtained using the bio-electrodes in Examples of the present invention.

### DESCRIPTION OF EMBODIMENTS

As described above, there was a need for developing a deep eutectic liquid that has high ionic conductivity and ensures safety upon contact with living bodies, a bio-electrode composition containing the deep eutectic liquid capable of promptly acquiring signals by being applied to the skin without leaving residues on the skin and stably obtaining biological signals for a long period, as well as a bio-electrode and a method for producing the bio-electrode.

The surface of skin releases ions of sodium, potassium, and calcium in accordance with heartbeat. It is necessary for a bio-electrode to convert the increase and decrease of these ions released from the skin to electrical signals. Accordingly, the bio-electrode requires a material that is excellent in ionic conductivity to transmit the increase and decrease of ions.

Ionic liquids are attracting attention as a material having ionic conductivity. Since these materials are present in a liquid state at room temperature, they exhibit high electroconductivity while having an organic structure. They also have ionic conductivity because of their rare property of being an ionic compound and at the same time being present in a liquid state at room temperature. However, ionic liquids are generally highly toxic to living organisms and therefore cannot be used for bio-electrodes.

Meanwhile, a material called a deep eutectic liquid has been known. Deep eutectic liquids are made by mixing a hydrogen bond donor compound with a hydrogen bond acceptor compound. Since the mixing of them causes a decrease of eutectic melting point, deep eutectic liquids are in a liquid state at room temperature. It is known that deep eutectic liquids, interestingly, exhibit properties similar to those of ionic liquids, while also offering a remarkably wide range of material choices. Accordingly, it has been discovered that selecting an ionic compound as the hydrogen bond acceptor compound for a deep eutectic liquid enables the formation of a deep eutectic liquid with ionic conductivity.

Biocompatibility is also important in the use of deep eutectic liquids for bio-electrodes. Therefore, possible materials for deep eutectic liquids include compounds used in living organisms such as sugars and amino acids, and medium- to high-molecular weight compounds such as oligomers and polymers. By selecting one of these materials, it is possible to obtain a deep eutectic liquid with no biological toxicity.

However, there have been very few reports on deep eutectic liquids formed of oligomers or polymers, with most previous reports focusing on deep eutectic liquids derived from monomers such as sugars and amino acids. Deep eutectic liquids have been considered for use in solvents or battery materials, both of which require low viscosity. Since medium- to high-molecular-weight deep eutectic liquids are expected to exhibit high viscosity, they have probably received little attention thus far. In addition, compared to monomers, polymers have lower flexibility in terms of molecular mobility and smaller capability for hydrogen bonding, which may also account for the lack of studies in this area.

Through extensive studies in light of the problems described above, the present inventors have interestingly found a deep eutectic liquid formed from an oligomer in which two or more monomer units are bonded, and also found that using such a deep eutectic liquid as a bio-electrode composition improves acquisition of biological signals, leading to the completion of the present invention.

That is, the deep eutectic liquid of the present invention is a mixture of a hydrogen bond donor compound and a hydrogen bond acceptor compound, wherein the hydrogen bond donor compound is a compound represented by the following general formula (1) having a structure in which 2 to 100 monomers having a hydroxy group are bonded, the hydrogen bond acceptor compound is a compound containing a monomer having a quaternary ammonium cation represented by the following general formulae (2) to (6) or a quaternary phosphonium cation represented by the following general formula (7), and the deep eutectic liquid is present in a liquid state at 25°C, 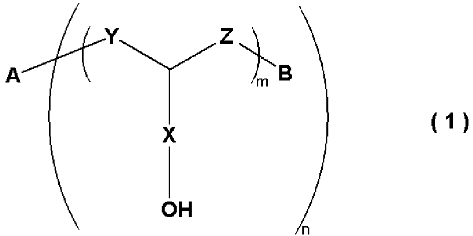 wherein X is a single bond, or a linear, branched, or cyclic divalent hydrocarbon group having 1 to 30 carbon atoms, which may be substituted with a heteroatom or intervened by a heteroatom; Y and Z each represent a linear, branched or cyclic divalent hydrocarbon group having 1 to 5 carbon atoms, which may be substituted with a heteroatom or intervened by a heteroatom; A and B each represent a hydrogen atom, a hydroxy group, an amino group, a halogen atom, or an alkyl group or an alkyl group with a terminal substituted with a siloxane that may be substituted with a heteroatom or intervened by a heteroatom; Y and Z may be identical to or different from each other; A and B may be identical to or different from each other; "m" is an integer of 1 to 100 and represents a repetition of chemical structural units, and "n" is an integer of 1 to 4 and represents a repetition of chemical structural units, provided that 2 ≤ m×n ≤ 100 is satisfied, 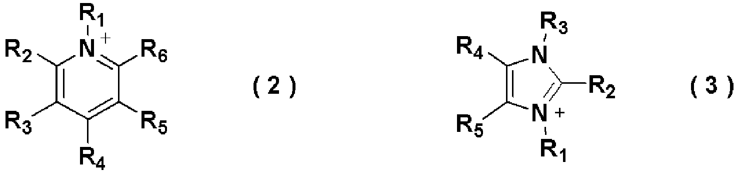 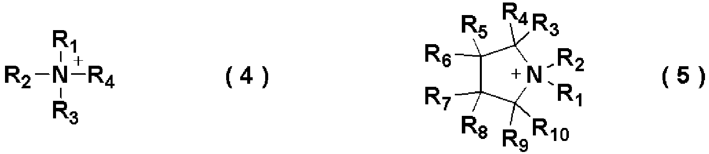 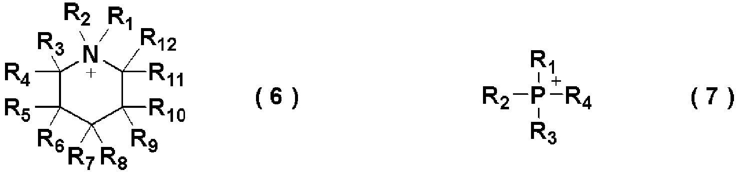 wherein R₁ to R₁₂ each represent a linear, branched, or cyclic monovalent hydrocarbon group having 1 to 30 carbon atoms that may be substituted with a heteroatom or intervened by a heteroatom and may form a zwitterion having an anionic portion, or a hydrogen atom, a hydroxy group, an amino group, a nitro group, or a halogen atom; R₁ to R₁₂ may be identical to or different from one another.

The deep eutectic liquid and the bio-electrode composition of the present invention exhibit excellent conductivity and biocompatibility, are lightweight, can be produced at low cost, and prevent significant reductions in conductivity and adhesion even when exposed to moisture or drying. Additionally, the deep eutectic liquid and the bio-electrode composition of the present invention are capable of forming a living body contact layer for bio-electrodes, which enables prompt signal acquisition upon application to the skin without leaving residues on the skin, and allows for long-term stable acquisition of biological signals.

Hereinafter, the present invention will be described in detail. However, the present invention is not limited thereto.

### [Deep Eutectic Liquid]

The deep eutectic liquid of the present invention is a mixture of a hydrogen bond donor compound and a hydrogen bond acceptor compound. The hydrogen bond donor compound is a compound represented by the following general formula (1) having a structure in which 2 to 100 monomers having a hydroxy group are bonded. The hydrogen bond acceptor compound is a compound containing a monomer having a quaternary ammonium cation represented by the following general formulae (2) to (6) or a quaternary phosphonium cation represented by the following general formula (7). The deep eutectic liquid is present in a liquid state at 25°C.

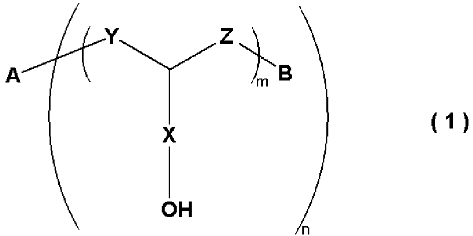

In the formula, X is a single bond, or a linear, branched, or cyclic divalent hydrocarbon group having 1 to 30 carbon atoms, which may be substituted with a heteroatom or intervened by a heteroatom; Y and Z each represent a linear, branched or cyclic divalent hydrocarbon group having 1 to 5 carbon atoms, which may be substituted with a heteroatom or intervened by a heteroatom; A and B each represent a hydrogen atom, a hydroxy group, an amino group, a halogen atom, or an alkyl group or an alkyl group with a terminal substituted with a siloxane that may be substituted with a heteroatom or intervened by a heteroatom; Y and Z may be identical to or different from each other; A and B may be identical to or different from each other; "m" is an integer of 1 to 100 and represents a repetition of chemical structural units, and "n**"** is an integer of 1 to 4 and represents a repetition of chemical structural units, provided that 2 ≤ m×n ≤ 100 is satisfied.

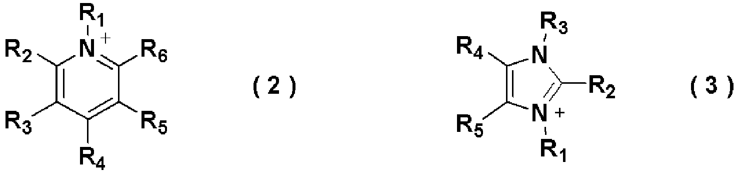

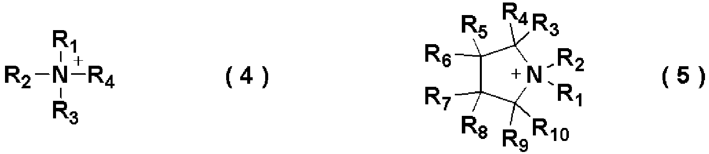

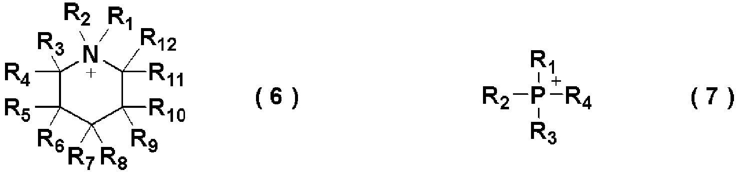

In the formula, R₁ to R₁₂ each represent a linear, branched, or cyclic monovalent hydrocarbon group having 1 to 30 carbon atoms that may be substituted with a heteroatom or intervened by a heteroatom and may form a zwitterion having an anionic portion, or a hydrogen atom, a hydroxy group, an amino group, a nitro group, or a halogen atom; R₁ to R₁₂ may be identical to or different from one another.

The hydrogen bond donor compound is a compound represented by the general formula (1), which has a structure in which 2 to 100 monomers having a hydroxy group are bonded. Specific examples of monomers having a hydroxy group include vinyl alcohol, glycerin, 2-hydroxyethyl acrylate, 4-hydroxybutyl acrylate, propylene glycol monoacrylate, 4-vinylphenol, 4-allylphenol, 4-vinyl-1,2-benzenediol, 4-allyl-1,2-benzenediol, and the like. In the present invention, each monomer having a hydroxy group is preferably glycerin, and the hydrogen bond donor compound is further preferably a polyglycerin-modified silicone represented by the following general formula (8) or (9). 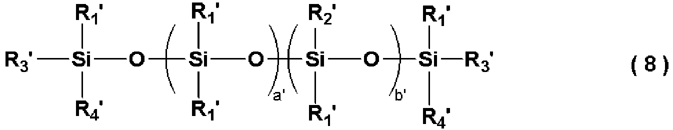 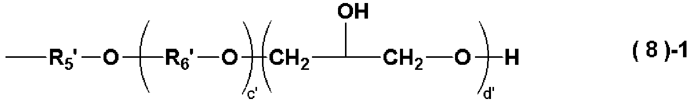 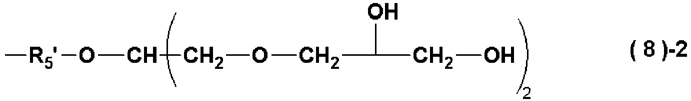 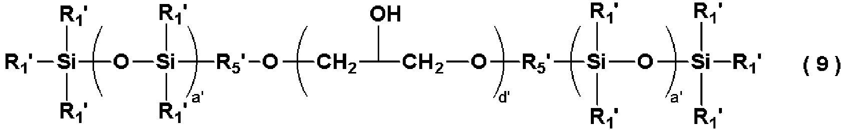 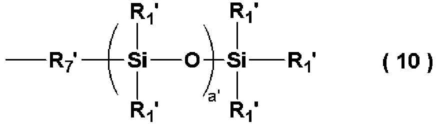 wherein R₁' is identical to or different from one another, and independently represents a hydrogen atom, a phenyl group, a linear or branched alkyl group having 1 to 50 carbon atoms, or a silicone chain represented by the general formula (10), and optionally contains an ether group; R₂' represents a group having a polyglycerin structure represented by the general formula (8)-1 or (8)-2; R₃' is identical to or different from one another, and independently represents the R₁' or the R₂'; R₄' is identical to or different from one another, and independently represents the R₁', the R₂', or an oxygen atom; when R₄' is an oxygen atom, two R₄' moieties may bond to each other to form an ether group and may form a ring together with silicon atoms to which they are bonded; "a'" is identical to or different from one another and represents 0 to 100, "b'" is 0 to 100, and a'+b' is 0 to 200, provided that, when "b'" is 0, at least one of R₃' is the R₂'; R₅' represents an alkylene group having 2 to 10 carbon atoms or an aralkylene group having 7 to 10 carbon atoms; R₆' and R₇' each represent an alkylene group having 2 to 6 carbon atoms; R₇' may be an ether group; "c'" is 0 to 20; and "d'" is 2 to 20.

The above polyglycerin-modified silicone has, for example, the following structure. 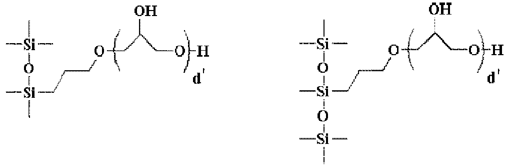 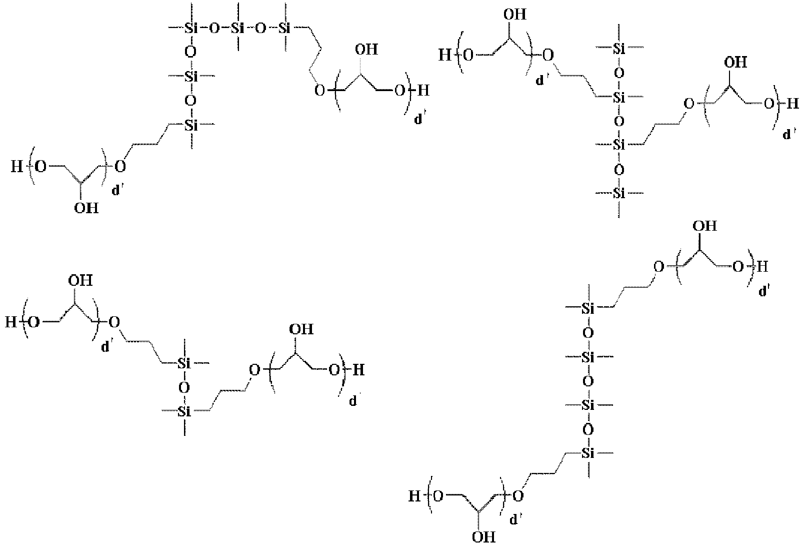 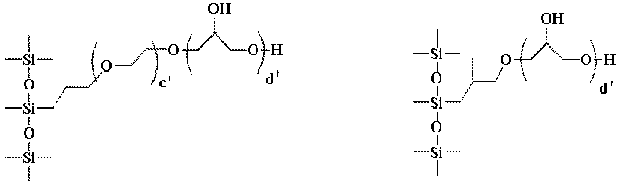 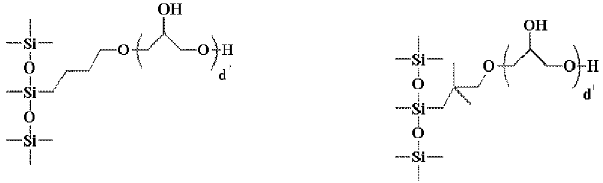 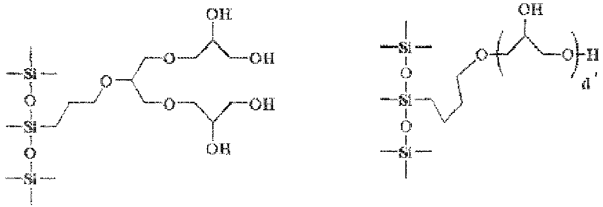 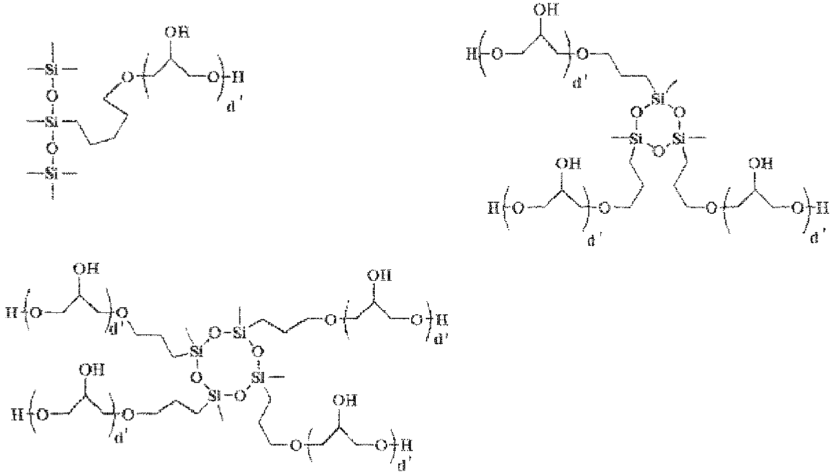 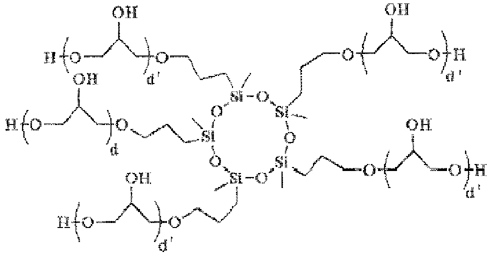 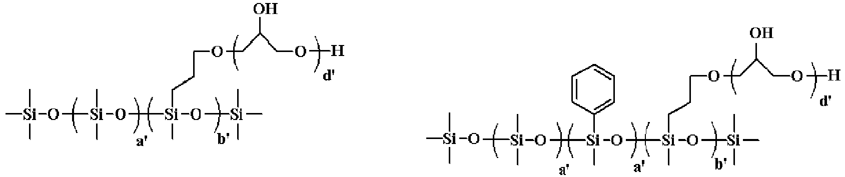 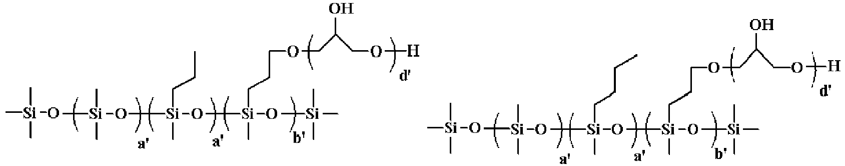 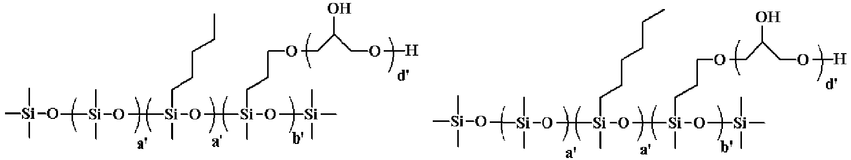 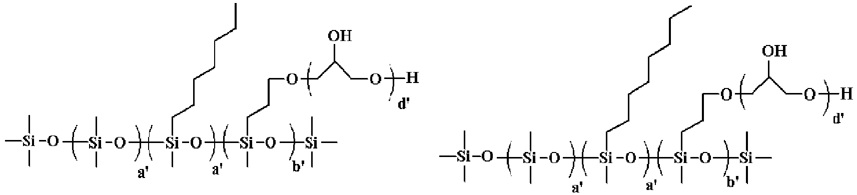 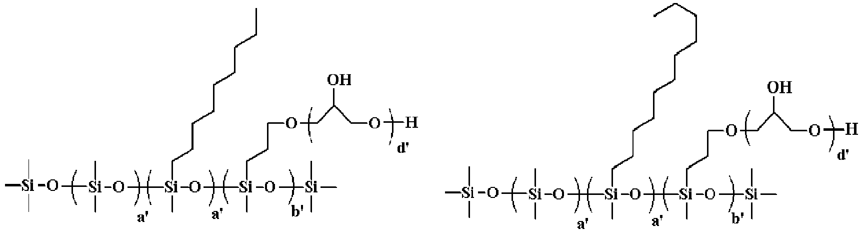 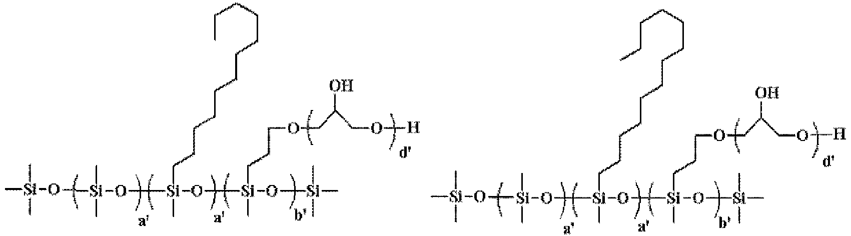 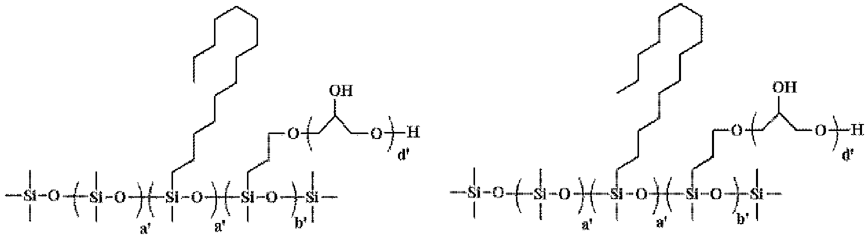 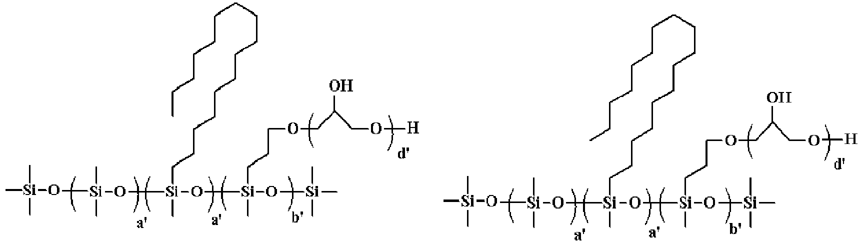 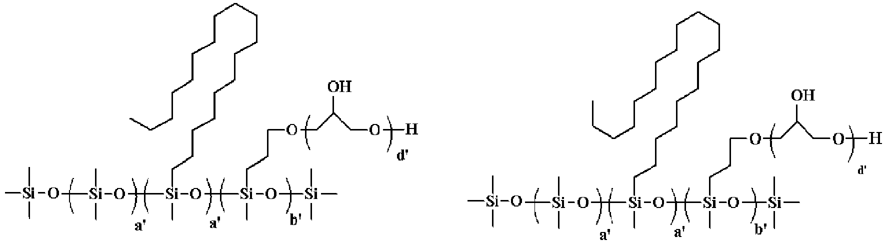 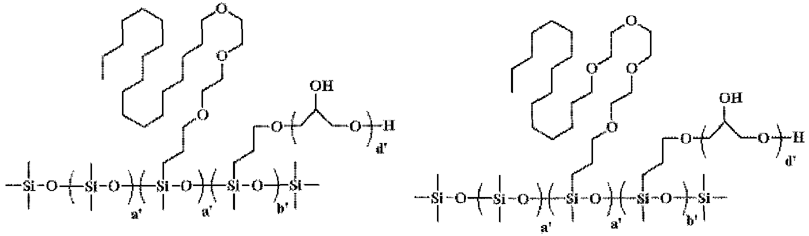 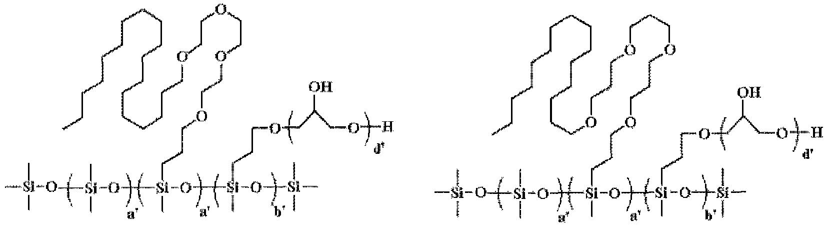 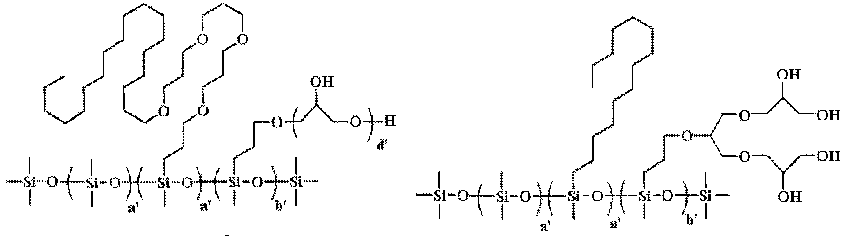 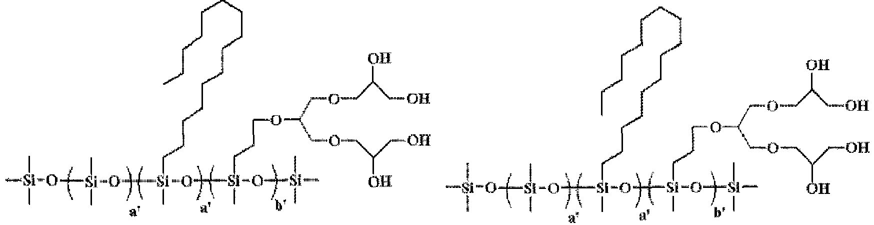 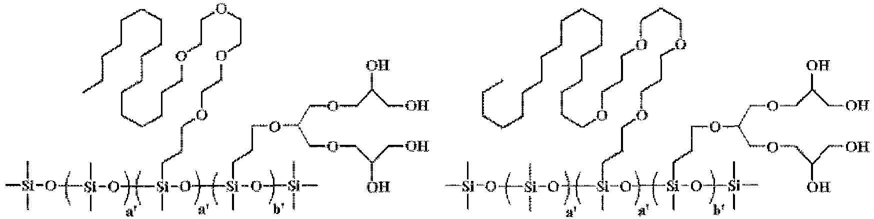 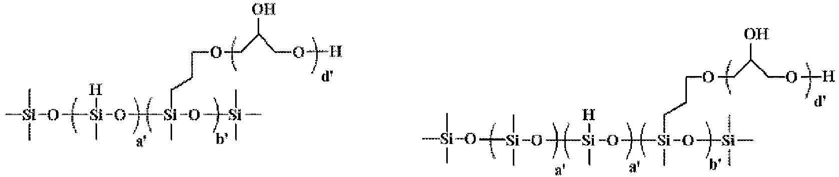 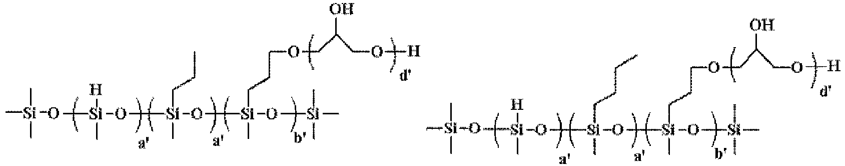 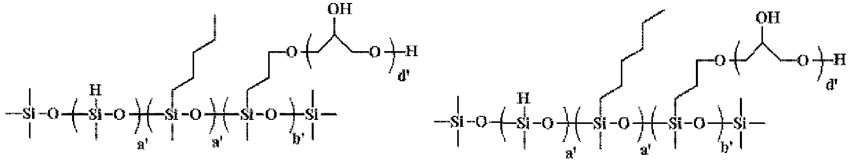 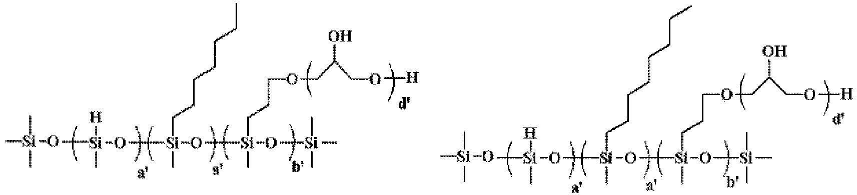 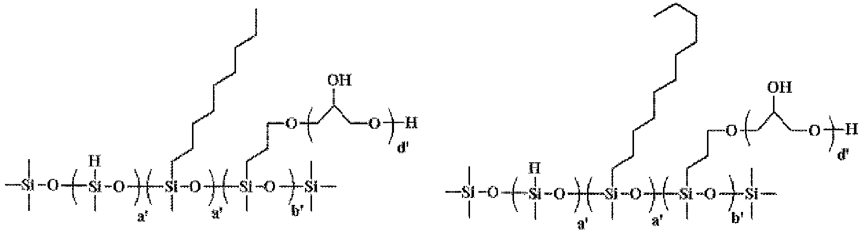 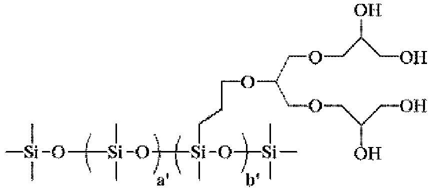 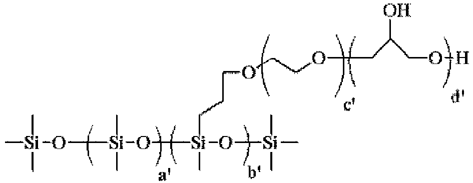 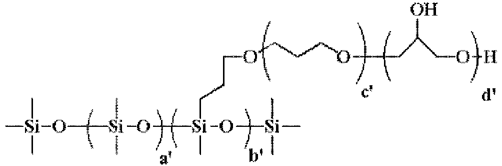 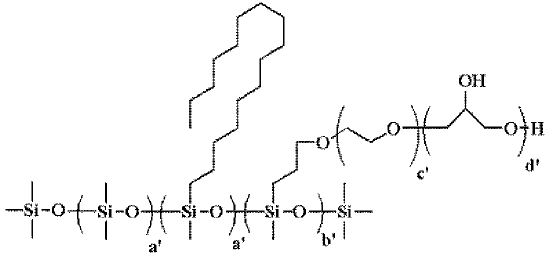 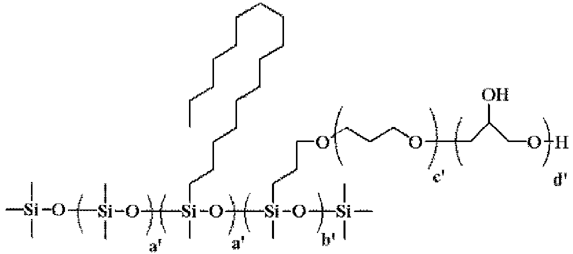 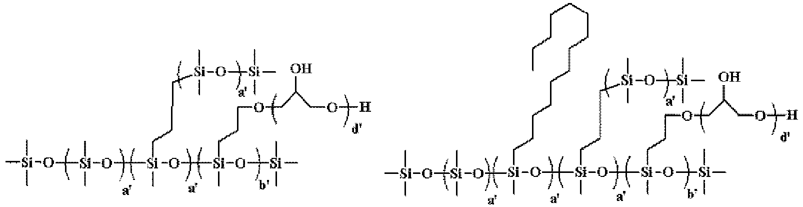 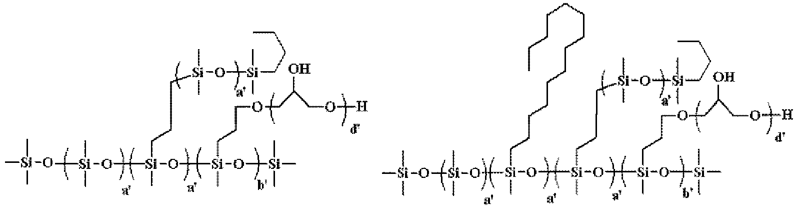 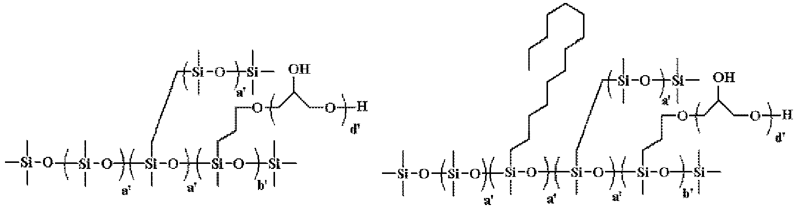 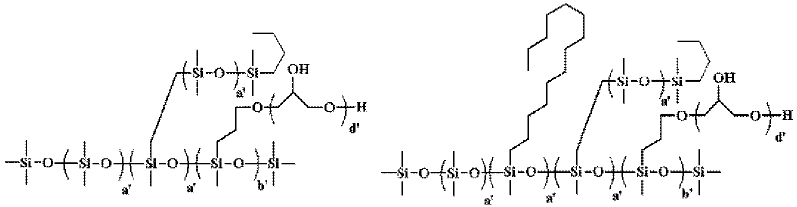 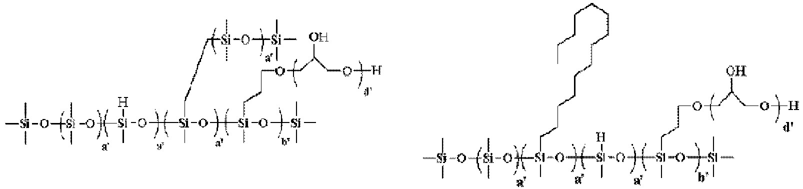 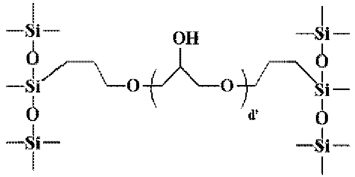 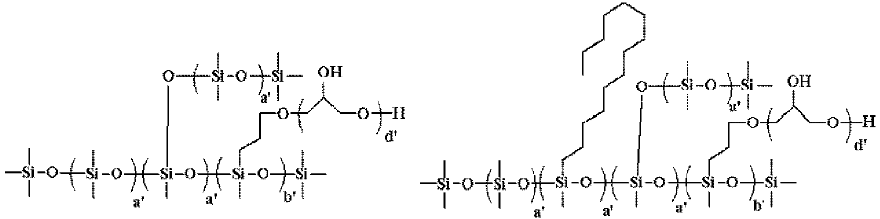 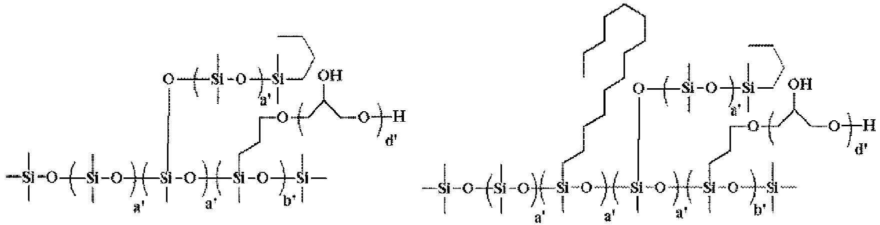 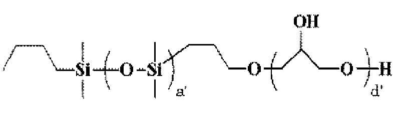 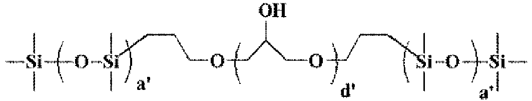 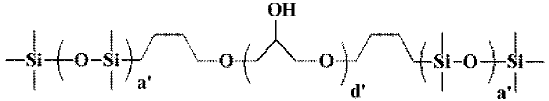 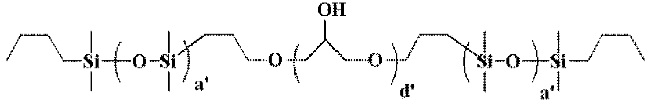 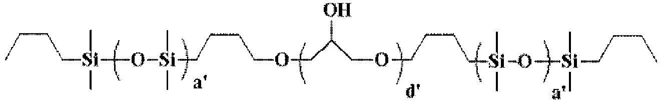 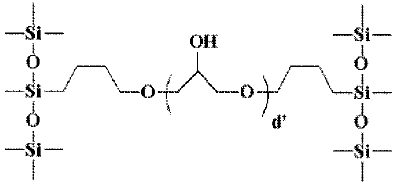 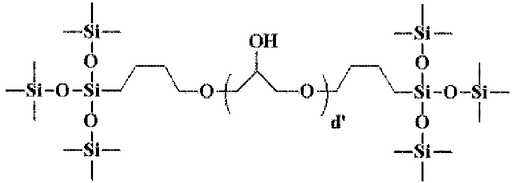 In the formulae, a', b', c', and d' are as defined above.

The hydrogen bond acceptor compound is a compound containing a monomer having a quaternary ammonium cation represented by the general formulae (2) to (6) or a quaternary phosphonium cation represented by the general formula (7). Specific examples of the compound include ammonium salts such as choline chloride, betaine, and tetrabutylammonium chloride; imidazole salts including such as 1-ethyl-3-methylimidazolium cation or 1-butyl-3-methylimidazolium cation; pyridinium salts such as 1-butylpyridinium chloride; pyrrolidinium salts such as 1-butyl-1-methylpyrrolidinium chloride; piperidinium salts such as 1-butyl-1-methylpiperidinium chloride; and phosphonium salts such as tributyl(methyl)phosphonium dimethyl phosphate. The biocompatible hydrogen bond acceptor compound is not limited to the compounds listed above, and, for example, their derivatives can also be considered candidates for deep eutectic liquid materials.

Deep eutectic liquids are generally obtained by mixing the above biocompatible hydrogen bond donor compound and the above biocompatible hydrogen bond acceptor compound, often in equimolar ratios. Although other molar ratios can also be observed, deep eutectic liquids typically exist within a molar ratio range of, for example, 1:1 to 10:1 or 1:1 to 5:1.

### [Bio-electrode Composition]

The present invention provides a bio-electrode composition containing the deep eutectic liquid described above.

### [Binder (A)]

The bio-electrode composition of the present invention may contain a binder (A) in addition to the above deep eutectic liquid. That is, the bio-electrode composition preferably contains the binder (A). For example, the bio-electrode composition of the present invention may include one or more resins selected from silicone-based resins, acrylic resins, and urethane-based resins. That is, it is preferable that the binder (A) be one or more resins selected from the group consisting of a silicone resin, a polyurethane resin, and a polyacrylic resin. By incorporating one or more resins selected from silicone-based resins, acrylic resins, and urethane-based resins, it is possible to provide a bio-electrode containing a living body contact layer with excellent stretchability.

The binder (A) that can be incorporated into the bio-electrode composition of the present invention may serve, for example, to prevent elution of the deep eutectic liquid and to impart adhesion. When the bio-electrode composition contains a conductive particle (B), which is described later, the binder (A) can retain the powder of (B). The binder (A) may be either a thermosetting resin or a photocurable resin, or a combination of both. In particular, the binder (A) is preferably one or more resins selected from silicone-based resins, acrylic resins, and urethane-based resins.

Examples of adhesive silicone-based resins include addition reaction-curable silicone-based resins and radical crosslinking reaction-curable silicone-based resins. As the addition reaction-curable silicone-based resin, it is possible to use one that contains diorganosiloxane having an alkenyl group(s), an MQ resin having R₃SiO_{0.5} and SiO₂ units, organohydrogenpolysiloxane having multiple SiH groups, a platinum catalyst, an addition-reaction inhibitor, and an organic solvent, for example, described in JP 2015-193803 A. As the radical crosslinking reaction-curable silicone-based resin, it is possible to use one that contains diorganopolysiloxane with or without an alkenyl group, an MQ resin having R₃SiO_{0.5} and SiO₂ units, organic peroxide, and an organic solvent, for example, described in JP 2015-193803 A. Here, R represents a substituted or unsubstituted monovalent hydrocarbon group having 1 to 10 carbon atoms.

It is also possible to use a polysiloxane-resin integrated compound that is formed by condensation reaction of an MQ resin and polysiloxane having silanol at the terminal or the side chain of the polymer. The MQ resin contains many silanols, and therefore adding MQ resin improves the adhesive strength; however, MQ resin does not bind to the polysiloxane at molecular level because of its incapability of crosslinking. The adhesive strength can be enhanced by integrating the polysiloxane and the resin as described above.

Further, the silicone-based resin may contain modified siloxane that has a group selected from an amino group, an oxirane group, an oxetane group, a polyether group, a hydroxy group, a carboxy group, a mercapto group, a methacryl group, an acryl group, a phenol group, a silanol group, a carboxylic anhydride group, an aryl group, an aralkyl group, an amide group, an ester group, and a lactone ring. The addition of the modified siloxane improves dispersibility of the deep eutectic liquid in the silicone resin. The modified siloxane may be modified at any part such as one terminal, both terminals, or a side chain of the siloxane.

As the adhesive acrylic resin, it is possible to use one having hydrophilic (meth)acrylic ester and hydrophobic long chain (meth)acrylic ester as the repeating units, for example, described in JP 2016-011338 A. In some cases, it is also possible to copolymerize (meth)acrylic ester having a functional group or (meth)acrylic ester having a siloxane bond.

As the adhesive urethane-based resin, it is possible to use one having a urethane bond with a polyether bond, a polyester bond, a polycarbonate bond, or a siloxane bond, for example, described in JP 2016-065238 A.

Further, in the bio-electrode composition of the present invention, the binder (A) preferably has high adhesion with respect to the conductive base material to prevent peeling of the living body contact layer from the conductive base material. In order to increase the adhesion to the conductive base material and the compatibility with the salt, a use of a resin with high polarity as the binder (A) is effective. Examples of such a resin include resin having one or more moieties selected from an ether bond, an ester bond, an amide bond, an imide bond, a urethane bond, a thiourethane bond, and a thiol group; a polyacrylic resin, a polyamide resin, a polyimide resin, a polyurethane resin, a polythiourethane resin; and the like. On the other hand, the living body contact layer comes in contact with a living body, thereby being susceptible to perspiration from the living body. Accordingly, in the bio-electrode composition of the present invention, the binder (A) preferably has high water repellency and is hardly hydrolyzed. To make the binder (A) highly water-repellent and hardly hydrolyzed, the use of a silicon atom-containing resin is effective.

The silicon atom-containing polyacrylic resin includes a polymer that has a silicone in the main chain and a polymer that has a silicon atom(s) in the side chain, either of which can be suitably used. As the polymer that has a silicone in the main chain, silsesquioxane, siloxane having a (meth)acrylpropyl group, or the like can be used. In this case, an addition of a photoradical generator allows the (meth)acryl moiety to polymerize to cure.

As the silicon atom-containing polyamide resin, it is possible to suitably use polyamide silicone resins or the like described in JP 2011-079946 A and US 5981680 A, for example. Such polyamide silicone resins can be synthesized by combining, for example, a silicone compound or non-silicone compound having amino groups at both terminals with a non-silicone compound or silicone compound having carboxy groups at both terminals.

It is also possible to use polyamic acid before cyclization thereof, which is obtained by reacting carboxylic anhydride and amine. The carboxy group of the polyamic acid may be crosslinked by using a crosslinking agent such as an epoxy-based crosslinking agent and an oxetane-based crosslinking agent. It is also possible to esterify the carboxy group with hydroxyethyl (meth)acrylate to perform photoradical crosslinking of the (meth)acrylate moiety.

As the silicon atom-containing polyimide resin, it is possible to suitably use polyimide silicone resins described in JP 2002-332305 A, for example. Although polyimide resins have very high viscosity, the viscosity can be decreased by blending a (meth)acrylic monomer as a solvent and as a crosslinking agent.

Examples of the silicon atom-containing polyurethane resins may include polyurethane silicone resins. Such polyurethane silicone resins can be crosslinked through urethane bond by blending a compound having isocyanate groups at both terminals and a compound having a hydroxy group(s) at the terminal(s), followed by heating. In this case, it is necessary to incorporate a silicon atom(s) (siloxane bond) in either or both of the compound having isocyanate groups at both terminals and the compound having a hydroxy group(s) at the terminal(s). Alternatively, polysiloxane and a urethane (meth)acrylate monomer can be blended and photo-crosslinked as described in JP 2005-320418 A. Further, it is also possible to photo-crosslink a polymer having both of a siloxane bond(s) and a urethane bond(s), with the terminal having a (meth)acrylate group(s). In particular, a polyurethane main chain having a silicone chain on a side chain as described in JP 2018-123304 A and JP 2019-070109 A is preferable because of the properties of high strength and high stretchability.

The silicon atom-containing polythiourethane resin can be obtained by reaction of a compound having a thiol group(s) and a compound having an isocyanate group(s), provided that either of them contains a silicon atom(s). It can also be photo-cured if a (meth)acrylate group(s) is contained at the terminal.

The silicone-based resin can be improved in adhesion to the conductive base material and compatibility with the salt by adding modified siloxane that has a group selected from an amino group, an oxirane group, an oxetane group, a polyether group, a hydroxy group, a carboxy group, a mercapto group, a methacryl group, an acryl group, a phenol group, a silanol group, a carboxylic anhydride group, an aryl group, an aralkyl group, an amide group, an ester group, and a lactone ring, in addition to the diorganosiloxane having an alkenyl group(s), the MQ resin having R₃SiO_{0.5} and SiO₂ units, and the organohydrogenpolysiloxane having multiple SiH groups.

In the bio-electrode composition of the present invention, the binder (A) is blended in an amount of preferably 0 to 2,000 parts by mass, more preferably 10 to 1,000 parts by mass, relative to 100 parts by mass of the deep eutectic liquid. Additionally, one kind of the binder (A) may be used alone, or two or more kinds of the binder (A) may be used in mixture.

As described later, the bio-electrode of the present invention contains a cured product of the bio-electrode composition including the deep eutectic liquid. By curing the bio-electrode composition, it is possible to improve the adhesion of the living body contact layer to both of skin and the conductive base material. The curing means is not particularly limited, and common means can be used, including a crosslinking reaction by either or both of heat and light, or with an acid catalyst or a base catalyst, for example. The crosslinking reaction can be performed, for example, by appropriately selecting methods described in "Kakyou han-nou handbook (handbook of crosslinking reaction)", Chapter 2, pages 51-371, Yasuharu Nakayama, Maruzen Publishing Co., Ltd. (2013).

The diorganosiloxane having an alkenyl group(s) and the organohydrogenpolysiloxane having multiple SiH groups can be crosslinked through an addition reaction with a platinum catalyst.

Examples of the platinum catalyst include platinum-based catalysts such as chloroplatinic acid, an alcohol solution of chloroplatinic acid, a reaction product of chloroplatinic acid and alcohol, a reaction product of chloroplatinic acid and an olefin compound, a reaction product of chloroplatinic acid and vinyl group-containing siloxane, a platinum-olefin complex, and a complex of platinum and vinyl group-containing siloxane; platinum group metal-based catalysts such as a rhodium complex and a ruthenium complex; and the like. These catalysts may be used after dissolved or dispersed in an alcohol solvent, a hydrocarbon-based solvent, or a siloxane solvent.

The platinum catalyst is added in an amount preferably within a range of 5 to 2,000 ppm, particularly preferably 10 to 500 ppm, relative to 100 parts by mass of the binder (A).

When the addition-curable silicone resin is used, an addition-reaction inhibitor may be added. This addition-reaction inhibitor is added as a quencher to prevent the action of the platinum catalyst in the solution and under a low temperature circumstance after forming the coating film and before heat curing the coating film. Specific examples of the addition-reaction inhibitor include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, 1,1,3,3-tetramethyl-1,3-divinyldisiloxane, and the like.

The addition-reaction inhibitor is added in an amount preferably within a range of 0 to 10 parts by mass, particularly preferably 0.05 to 3 parts by mass, relative to 100 parts by mass of the binder (A).

Methods for performing photocuring include a method that uses resins with (meth)acrylate or olefin terminal or add a crosslinking agent with (meth)acrylate, olefin, or thiol terminal while adding a photoradical initiator that generates radicals upon exposure to light, and a method using resins or crosslinking agents containing oxirane, oxetane, or vinyl ether groups while adding a photoacid generator that produces acids upon exposure to light.

Examples of the photoradical generator may include acetophenone, 4,4'-dimethoxybenzyl, benzyl, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxole-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthene-9-one, diphenyl(2,4,6-trimethylbenzoyl)phosphine oxide (BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The curing can also be performed by adding a heat decomposition radical generator. Examples of the heat decomposition radical generator may include 2,2'-azobis(isobutyronitrile), 2,2'-azobis(2,4-dimethylvaleronitrile), 2,2'-azobis(2-methylbutyronitrile), 2,2'-azobis(methylpropionamidine) hydrochloride, 2,2'-azobis[2-(2-imidazolin-2-yl)propane]hydrochloride, 2,2'-azobis(4-methoxy-2,4-dimethylvaleronitrile), 2,2'-azobis(2-methylbutyronitrile), 2,2'-azobis(cyclohexane-1-carbonitrile), 1[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutylate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, dicumyl peroxide, and the like.

Examples of the photoacid generator may include sulfonium salts, iodonium salts, sulfonyl diazomethane, N-sulfonyl oxyimides, oxime-O-sulfonate acid generators, and the like. Specific examples of the photoacid generator include, for example, those described in paragraphs [0122] to [0142] of JP 2008-111103 A and those described in JP 2009-080474 A.

Note that, the radical generator and the photoacid generator are added in an amount preferably within a range of 0.1 to 50 parts by mass, relative to 100 parts by mass of the binder (A).

Among these, the binder (A) particularly preferably contains one of diorganosiloxane having an alkenyl group, organohydrogenpolysiloxane having an SiH group, and a silicone resin having an SiO₂ unit and an RₓSiO_{(4-x)/2} unit, wherein R represents a substituted or unsubstituted monovalent hydrocarbon group having 1 to 10 carbon atoms, and "x" represents a number in a range of 2.5 to 3.5. The binder (A) thus described is compatibilized with the deep eutectic liquid described above to prevent elution of the salt, and provides more enhanced adhesion to the living body contact layer.

### [Conductive Particle (B)]

It is also preferable that the bio-electrode composition described above contain conductive particle (B). Specific examples of the conductive particles (B) may include carbon powder, metal powder, and the like. That is, the conductive particles (B) preferably contain one or more selected from the group consisting of carbon powder, gold, silver, silver chloride, platinum, aluminum, magnesium, tin, tungsten, iron, copper, nickel, stainless steel, chromium, titanium, molybdenum, ruthenium, and indium.

### [Metal Powder]

The bio-electrode composition of the present invention may also include metal powder selected from gold, silver, silver chloride, platinum, copper, tin, titanium, nickel, aluminum, magnesium, tungsten, iron, stainless steel, molybdenum, ruthenium, chromium, and indium to enhance electronic conductivity. The metal powder is added in an amount preferably within a range of 1 to 50 parts by mass, relative to 100 parts by mass of the binder (A).

As the kind of the metal powder, gold, silver, and platinum are preferable from the viewpoint of electric conductivity; and silver, copper, tin, titanium, nickel, aluminum, tungsten, molybdenum, ruthenium, and chromium are preferable from the viewpoint of cost. From the viewpoint of biocompatibility, noble metals are preferable. From comprehensive viewpoint including the above, silver is most preferable.

Shapes of the metal powder may include a spherical shape, a disk shape, a flaky shape, a needle shape, and the like. However, addition of flaky powder is preferable as it provides the highest electric conductivity. The metal powder is preferably a flake having relatively lower density and larger specific surface area with a size of 100 µm or less, a tapped density of 5 g/cm³ or less, and a specific surface area of 0.5 m²/g or more. The size is determined by laser diffraction, the tapped density (bulk density after compaction) is measured using a Powder Tester PT-X available from Horikawa Micron Corporation, and the specific surface area is measured based on the gas adsorption method for powder surface area measurement, using liquid nitrogen adsorption at 77K with a Microtrac BEL BELSORP-max.

### [Carbon Powder]

Carbon powder can be added as the conductive particles (B). Examples of the carbon powder (carbon material) may include carbon black, graphite, carbon nanotube, carbon fiber, and the like. The carbon nanotube may be either single layer or multilayer, and the surface may be modified with an organic group(s). The carbon material is added in an amount preferably within a range of 1 to 50 parts by mass, relative to 100 parts by mass of the binder (A). The carbon powder is preferably one or both of carbon black and carbon nanotube.

### [Organic Solvent]

Further, the bio-electrode composition of the present invention may contain an organic solvent. Specific examples of the organic solvent may include aromatic hydrocarbon-based solvents such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon-based solvents such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin; ketone solvents such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, and methyl n-pentyl ketone; alcohol solvents such as 3-methoxybutanol, 3-methyl-3-methoxybutanol, 1-methoxy-2-propanol, and 1-ethoxy-2-propanol; ether solvents such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, di-sec-pentyl ether, di-tert-amyl ether, di-n-hexyl ether, and anisole; ester solvents such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactone solvents such as γ-butyrolactone; and the like.

Further, the bio-electrode composition of the present invention may contain water as a solvent.

The organic solvent and water are added in an amount preferably within a range of 70 to 150 parts by mass, relative to 100 parts by mass of the binder (A).

### [Tackifier]

Further, the bio-electrode composition of the present invention may also contain a tackifier to provide adhesion to the living body. Examples of such a tackifier may include silicone resins, non-crosslinked siloxanes, non-crosslinked poly(meth)acrylates, non-crosslinked polyethers, and the like. The tackifier is added in an amount preferably within a range of 1 to 20 parts by mass, relative to 100 parts by mass of the binder (A).

### [Crosslinking Agent]

The bio-electrode composition of the present invention may contain an epoxy-based crosslinking agent. The crosslinking agent in this case is a compound having multiple epoxy groups or oxetane groups in one molecule. The crosslinking agent is added in an amount preferably within a range of 1 to 30 parts by mass, relative to 100 parts by mass of the binder (A).

### [Crosslinking Catalyst]

The bio-electrode composition of the present invention may also contain a crosslinking catalyst for crosslinking the epoxy groups or the oxetane groups. As this crosslinking catalyst, for example, those described in paragraphs [0027] to [0029] of JP 2019-503406 A can be used. The crosslinking catalyst is added in an amount preferably within a range of 0.01 to 10 parts by mass, relative to 100 parts by mass of the binder (A).

### [Ionic Additive]

The bio-electrode composition of the present invention may contain an ionic additive to enhance ionic conductivity. In consideration of biocompatibility, examples of the ionic additive may include sodium chloride, potassium chloride, calcium chloride, saccharin, acesulfame potassium, and salts disclosed in JP 2018-044147 A, JP 2018-059050 A, JP 2018-059052 A, and JP 2018-130534 A.

It is also preferable that the bio-electrode composition contain glycerin. The glycerin is added in an amount preferably within a range of 1 to 30 parts by mass, relative to 100 parts by mass of the binder (A).

### [Compound (C) having Polyglycerin Structure]

The bio-electrode composition of the present invention may contain a compound (C) having a polyglycerin structure to enhance ionic conductivity and reduce contact impedance with the skin. Examples of the compound (C) having a polyglycerin structure include polyglycerin (a dimer to decamer of glycerin) and polyglycerin-modified silicones (polyglyceryl-3 polydimethylsiloxyethyl dimethicone and lauryl polyglyceryl-3 polydimethylsiloxyethyl dimethicone). The compound (C) is added in an amount preferably within a range of 1 to 30 parts by mass, relative to 100 parts by mass of the binder (A).

As described above, the bio-electrode composition of the present invention is capable of forming a living body contact layer for a bio-electrode, which exhibits excellent electric conductivity and biocompatibility, is lightweight, can be produced at low cost, prevents significant reduction in electric conductivity even when exposed to moisture or drying, and enables prompt signal acquisition upon application to the skin without leaving residues on the skin. The living body contact layer, which is formed by curing the bio-electrode composition of the present invention, can exhibit excellent electric conductivity and thus efficiently transmit electrical signals from a living body, such as the skin, to a device. Further, since the living body contact layer can exhibit excellent biocompatibility, it can prevent allergic reactions even when worn on the skin for a long period. Further, by adding a conductivity improver such as a carbon material, the electric conductivity can be further improved. Further, by combining the bio-electrode composition of the present invention with a resin having adhesion and stretchability, it is possible to produce a bio-electrode with particularly high adhesive strength and excellent stretchability. Furthermore, the bio-electrode composition of the present invention can be improved in stretchability and adhesiveness with respect to the skin by adding additives or the like. Furthermore, by appropriately adjusting the type and amount of the deep eutectic liquid used in the bio-electrode composition of the present invention, the composition of the binder (A), the amount of the conductive particles (B), and the thickness of the living body contact layer, it is also possible to control the stretchability and adhesion.

### <Bio-Electrode>

Further, the present invention provides a bio-electrode containing a conductive base material and a living body contact layer formed on the conductive base material. The living body contact layer contains a cured product of the bio-electrode composition described above.

The bio-electrode of the present invention is described below in detail with reference to drawings; however, the present invention is not limited to the bio-electrodes described below.

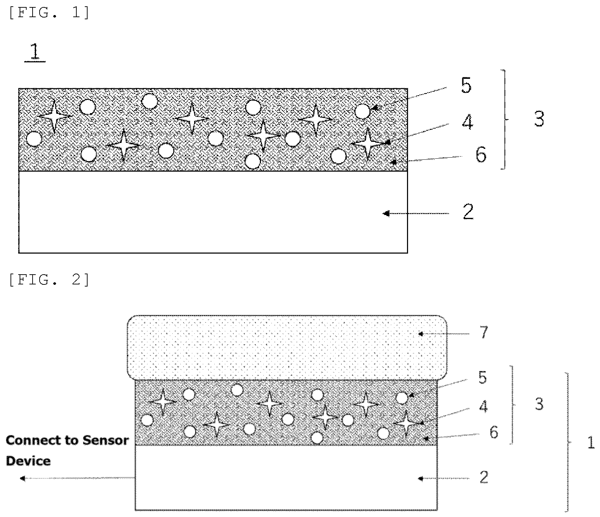

FIG. 1 is a schematic cross-sectional view showing an example of a bio-electrode of the present invention. In FIG. 1, a bio-electrode 1 has a conductive base material 2 and a living body contact layer 3 formed on the conductive base material 2. The living body contact layer 3 is a layer in which a deep eutectic liquid 5 and conductive particles 4 are dispersed in a resin 6. The resin 6 is, for example, the compound (C) having a polyglycerin structure and the binder resin (A) described above. The living body contact layer 3 is an example of a cured product of the bio-electrode composition of the present invention.

When using such a bio-electrode 1 of FIG. 1, as shown in FIG. 2, the living body contact layer 3 (i.e., a layer in which the deep eutectic liquid 5 and the conductive particles 4 are dispersed in the resin 6) is brought into contact with a living body 7 to extract electrical signals from the living body 7 by the deep eutectic liquid 5 and the conductive particles 4. The electrical signals thus extracted are transmitted through the conductive base material 2 to a sensor device or the like (not shown). As described above, the living body contact layer containing a cured product of the bio-electrode composition of the present invention is capable of ensuring both electric conductivity and biocompatibility by using the deep eutectic liquid described above, and stably obtaining electrical signals from the skin at high sensitivity because the contact area with the skin is kept constant due to the adhesiveness thereof. In particular, as previously described, the bio-electrode composition of the present invention is capable of forming a living body contact layer that can promptly acquire signals by being applied to the skin. Accordingly, the bio-electrode 1 of FIG. 1 can promptly acquire signals by being applied to the living body 7, as shown in FIG. 2.

Hereinafter, each component of the bio-electrode of the present invention is described further in detail.

### [Conductive Base Material]

The bio-electrode of the present invention has a conductive base material. The conductive base material is usually connected electrically with a sensor device or the like, and conducts electrical signals picked from a living body through the living body contact layer to the sensor device or the like.

The conductive base material is not particularly limited, as long as it has electric conductivity. The conductive base material preferably contains one or more selected from the group consisting of gold, silver, silver chloride, platinum, aluminum, magnesium, tin, tungsten, iron, copper, nickel, stainless steel, chromium, titanium, and carbon, for example.

Further, the conductive base material is not particularly limited and may be a rigid conductive substrate or the like, or a conductive film having flexibility, a substrate having a stretchable film coated with a conductive paste, a fabric with a surface coated with a conductive paste, or a fabric impregnated with a conductive polymer. The conductive base material may be flat, uneven, or mesh-form of woven metal wires, which can be appropriately selected in accordance with the use of the bio-electrode, and so forth. Among these, considering its application onto the skin, a substrate having a stretchable film or fabric coated with a conductive paste is preferred. Examples of the stretchable film may include polyurethane and polyester films. As the conductive paste, a mixture of a conductive powder such as carbon, silver, gold, or copper with a solvent in a stretchable resin, such as polyurethane, polyester, silicone, or nitrile resin, is used.

### [Living Body Contact Layer]

The bio-electrode of the present invention has a living body contact layer formed on the conductive base material. This living body contact layer is a part to be actually in contact with a living body when the bio-electrode is used. The living body contact layer has electric conductivity. The living body contact layer is an adhesive resin layer that contains, in addition to the aforementioned deep eutectic liquid, optionally, additives such as the binder (A), the conductive particles (B), glycerin, and the compound (C) having a polyglycerin structure.

The living body contact layer preferably has an adhesive strength in a range of 0.5 N/24 mm or more and 20 N/24 mm or less. The adhesive strength is commonly measured by the method described in JIS Z 0237, in which a metal substrate such as a stainless steel (SUS) substrate or a polyethylene terephthalate (PET) substrate can be used as a base material. However, alternatively, human skin can be used in the measurement. Human skin has lower surface energy than metals and various plastics, and the surface energy is as low as that of Teflon (registered trademark). Therefore, human skin has difficulty to be adhered to.

The living body contact layer of the bio-electrode has a thickness of preferably 1 µm or more and 5 mm or less, more preferably 2 µm or more and 3 mm or less. As the living body contact layer is thinner, the adhesive strength lowers, but the flexibility is improved, the weight decreases and thus the compatibility with skin is improved. The thickness of the living body contact layer can be selected based on the balance of adhesiveness and texture to the skin.

The bio-electrode of the present invention may be additionally provided with an adherent film on the living body contact layer as in conventional bio-electrodes (e.g., the bio-electrode described in JP 2004-033468 A) in order to prevent the bio-electrode peeling off from a living body during the use. When the adherent film is provided additionally, the adherent film may be formed by using an adherent film material such as an acrylic, a urethane, and a silicone material. In particular, the silicone adherent film material is suitable because of: the high oxygen permeability, which enables dermal respiration while the film is attached to the skin; the high water repellency, which suppresses lowering of adhesion due to perspiration; and further the low irritation to skin. It is to be noted that the living body contact layer in the bio-electrode of the present invention does not necessarily require this adherent film that is provided additionally, because peeling off from a living body can be prevented by adding a tackifier to the living body contact layer or using a resin having good adhesiveness to the living body as described above.

When the bio-electrode of the present invention is used for a wearable device, wiring between the living body contact layer and a sensor device, and other components are not particularly limited. For example, it is possible to use those described in JP 2004-033468 A.

As described above, the bio-electrode of the present invention contains the living body contact layer containing a cured product of the bio-electrode composition of the present invention described above. The bio-electrode of the present invention thus can have the living body contact layer that exhibits excellent electric conductivity and biocompatibility, is lightweight, and can be produced at low cost, prevents significant reduction in electric conductivity even when exposed to moisture or drying, and enables prompt signal acquisition upon application to the skin without leaving residues on the skin. The living body contact layer of the bio-electrode of the present invention can exhibit excellent electric conductivity and thus efficiently transmit electrical signals from a living body, such as the skin, to a device. Further, since the living body contact layer can exhibit excellent biocompatibility, it can prevent allergic reactions even when worn on the skin for a long period. Further, the electric conductivity of the living body contact layer of the bio-electrode of the present invention can be further improved by adding metal powder. Further, by combining the living body contact layer of the present invention with a resin having adhesion and stretchability, it is possible to produce a bio-electrode with particularly high adhesive strength and excellent stretchability. Furthermore, the living body contact layer can be improved in stretchability and adhesiveness with respect to the skin by adding additives or the like to the living body contact layer. Furthermore, by appropriately adjusting the type and amount of the deep eutectic liquid of the present invention, the composition of the binder (A), the amount of the conductive particles (B), and the thickness of the living body contact layer, it is also possible to control the stretchability and adhesion of the living body contact layer. Accordingly, the bio-electrode of the present invention as described above is particularly suitable as a bio-electrode used for medical wearable devices.

### <Method for Producing Bio-Electrode>

Further, the present invention provides a method for producing a bio-electrode having a conductive base material and a living body contact layer formed on the conductive base material. The method includes applying the bio-electrode composition described above onto the conductive base material, and curing the bio-electrode composition to form the living body contact layer.

Further, the present invention provides another method for producing a bio-electrode having a conductive base material and a living body contact layer formed on the conductive base material. The method includes applying the bio-electrode composition described above onto a release substrate, followed by curing of the bio-electrode composition, and patterning of the cured product, and transferring the patterned product onto the conductive base material to form the living body contact layer.

Note that, the conductive base material, the living body contact layer, and the like used in the method for producing a bio-electrode of the present invention may be similar to those described above.

### [Release Substrate]

For example, fluorine-based release films (SSIA and FSD5) available from Nippa Corporation can be used as the release substrate for the method for producing the bio-electrode of the present invention. The bio-electrode composition of the present invention is applied onto such a release substrate and cured.

The method for forming the living body contact layer on the conductive base material is not particularly limited; however, suitable methods include dip coating, spray coating, spin coating, roll coating, flow coating, doctor coating, screen printing, flexographic printing, gravure printing, stencil printing, and inkjet printing, for example. Other methods include solid film formation with comma coaters and slit coaters, pattern printing with screen printing and stencil printing, and the like.

The curing method of the bio-electrode composition is not particularly limited, and may be appropriately selected depending on the types of the deep eutectic liquid and the binder (A) contained in the bio-electrode composition. However, it is preferable to cure the bio-electrode composition using, for example, either heat or light, or both. Further, the bio-electrode composition described above can also be cured by adding thereto a catalyst in advance to generate acid or base, which causes a crosslinking reaction.

In case of heating, the heating temperature is not particularly limited and may be appropriately selected depending on the types of the deep eutectic liquid and the binder (A) contained in the bio-electrode composition. However, a temperature of approximately 50 to 250°C is preferable, for example.

When the heating and light irradiation are combined, it is possible to perform the heating and the light irradiation simultaneously, or first perform the light irradiation and then the heating, or first perform the heating and then the light irradiation. It is also possible to perform air-drying to evaporate the solvent before heating the coating film.

In the case of a solid film, the film is laminated with release films, and the laminated film is cut into an arbitrary pattern using scissors or a cutter. Then, one of the release films is peeled off, and the patterned product is transferred and attached onto a conductive base material. Then, the second release film is peeled off to form a living body contact layer.

In the case of pattern printing such as screen printing or stencil printing, an arbitrary pattern can be printed, allowing the patterned product to be transferred and attached onto a conductive base material to form a living body contact layer.

Before applying the living body contact layer of the present invention to the skin, the sensitivity of the biological signal can be improved by wiping the skin with gauze, absorbent cotton, nonwoven fabric, or the like containing water or alcohol, thereby removing sebum from the skin and moistening the skin. If the skin is dry, the release of ions from the skin does not occur. By moistening the skin or the living body contact layer, the release of ions from the skin is facilitated, thereby enhancing the sensitivity of the biological signals. It is preferable that the substance impregnated into absorbent cotton, nonwoven fabric, gauze, or the like is water or a water-soluble alcohol, such as ethanol, glycerin, ethylene glycol, or diethylene glycol, that contains water.

As described above, the method for producing a bio-electrode of the present invention is capable of easily and inexpensively producing the bio-electrode of the present invention that exhibits excellent electric conductivity and biocompatibility, is lightweight, and prevents significant reduction in electric conductivity even when exposed to moisture or drying, and also enables prompt signal acquisition upon application to the skin without leaving residues on the skin.

### EXAMPLE

The present invention is specifically described below with reference to Examples and Comparative Examples. However, the present invention is not limited to these Examples. Note that, "Me" represents a methyl group, and "Vi" represents a vinyl group.

### [Deep Eutectic Liquid]

The hydrogen bond donor compound and the hydrogen bond acceptor compound incorporated in the deep eutectic liquids in the Examples are listed below.
Hydrogen Bond Donor Compound
Polyglycerin-modified silicone: KF-6100, available from Shin-Etsu Chemical Co., Ltd.
Polyglycerin: Polyglycerin #310
   Polyglycerin #500
   Polyglycerin #750
available from Sakamoto Yakuhin Kogyo Co., Ltd.
Hydrogen Bond Acceptor Compound
Quaternary ammonium cation-containing monomer:
   Choline chloride
   Betaine anhydrous
available from Tokyo Chemical Industry Co., Ltd.

Further, the deep eutectic liquids 1 to 9 incorporated in the bio-electrode compositions of Examples were synthesized as follows.

In a reaction container, a hydrogen bond donor compound and a hydrogen bond acceptor compound were mixed at a predetermined molar ratio and heated in an oven at 125°C for 3 to 6 hours. The deep eutectic liquids 1 to 9 synthesized as above are shown below. The obtained deep eutectic liquids 1 to 9 all was in a liquid state at 25°C.

**[Table 1]**

| | Hydrogen Bond Donor Compound 1 | Hydrogen Bond Donor Compound 2 | Hydrogen Bond Acceptor Compound 1 | Molar Ratio |
|---|---|---|---|---|
| Deep Eutectic Liquid 1 | KF-6100 | - | Choline Chloride | 3:1 |
| Deep Eutectic Liquid 2 | KF-6100 | - | Choline Chloride | 5:1 |
| Deep Eutectic Liquid 3 | KF-6100 | - | Choline Chloride | 10:1 |
| Deep Eutectic Liquid 4 | KF-6100 | - | Betaine Anhydrous | 5:1 |
| Deep Eutectic Liquid 5 | KF-6100 | - | Betaine Anhydrous | 10:1 |
| Deep Eutectic Liquid 6 | Polyglycerin #310 | - | Choline Chloride | 1:1 |
| Deep Eutectic Liquid 7 | Polyglycerin #500 | - | Choline Chloride | 1:1 |
| Deep Eutectic Liquid 8 | Polyglycerin #750 | - | Choline Chloride | 1:1 |
| Deep Eutectic Liquid 9 | KF-6100 | Polyglycerin #310 | Choline Chloride | 1:1:1 |

### [Binder Resin (A)]

Siloxane compounds 1 to 4, which were incorporated as silicone-based resins to the bio-electrode compositions of Examples and Comparative Examples, are shown below. The viscosity was measured using a cone and plate viscometer (available from Brookfield), with a cone rotor of R = 12 mm, a cone angle of 3°, and a rotation rate of 10 rpm.

### (Siloxane Compound 1)

Siloxane compound 1 was vinyl group-containing polydimethylsiloxane, which has an alkenyl group-content of 0.007 mol/100 g and a viscosity of 27,000 mPa·s for the 30% solution in toluene. In siloxane compound 1, the terminals of molecular chain are capped with SiMe₂Vi groups.

### (Siloxane Compound 2)

Siloxane compound 2 was a 60% solution of polysiloxane of MQ resin having an Me₃SiO_{0.5} unit and an SiO₂ unit (Me₃SiO_{0.5} unit/SiO₂ unit=0.8) in toluene.

### (Siloxane Compound 3)

Siloxane compound 3 was polydimethylsiloxane-bonded MQ resin obtained by heating the following solution under reflux for 4 hours, followed by cooling. The solution contains 40 parts by mass of vinyl group-containing polydimethylsiloxane, which has an alkenyl group-content of 0.007 mol/100 g and a viscosity of 42,000 mPa·s for the 30% solution in toluene, in which the terminals of molecular chain are capped with OH; 100 parts by mass of 60% solution of polysiloxane of MQ resin having an Me₃SiO_{0.5} unit and an SiO₂ unit (Me₃SiO_{0.5} unit/SiO₂ unit=0.8) in toluene; and 26.7 parts by mass of toluene.

### (Siloxane Compound 4)

As methylhydrogensilicone oil, KF-99 available from Shin-Etsu Chemical Co., Ltd. was used.

Acrylic polymer 1 incorporated as an acrylic resin into the bio-electrode compositions of the Examples and Comparative Examples is shown below. Further, the molecular weight (Mw) and the dispersity (Mw/Mn) of the obtained polymer were determined by gel permeation chromatography (GPC) using tetrahydrofuran (THF) as a solvent. The measurement temperature at this time was 40°C.

### Acrylic polymer 1

Mw = 655,000
Mw/Mn = 2.32

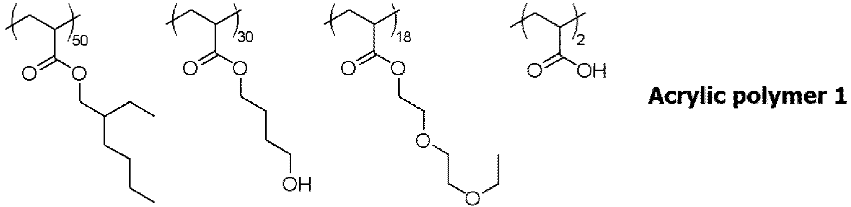

The repeating number in each formula shows the average value.

Urethane resins 1 to 3, which were incorporated as urethane-based resins into the bio-electrode compositions of Examples and Comparative Examples, are shown below. Further, the molecular weight (Mw) and the dispersity (Mw/Mn) of the obtained resin were determined by gel permeation chromatography (GPC) using tetrahydrofuran (THF) as a solvent. The measurement temperature at this time was 40°C. 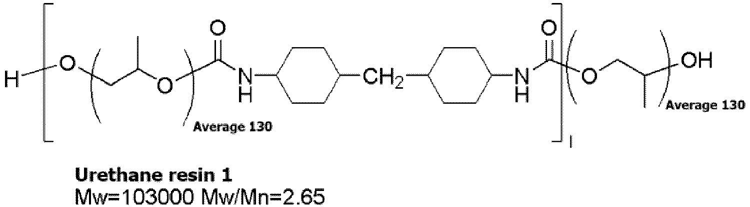 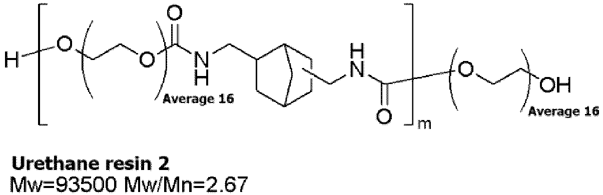 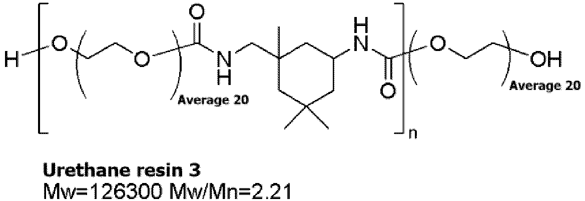 The repeating number in each formula shows the average value; and "l" is 12, "m" is 100, and "n" is 110.

### [Conductive Particle (B)]

Conductive Particles (B), which were incorporated into the bio-electrode compositions of Examples and Comparative Examples, are shown below.

### Metal Powder

Silver powder: silver flake having a diameter of 10 µm, available from Sigma-Aldrich Co. LLC
Gold powder: gold powder having a diameter of 10 µm or less, available from Sigma-Aldrich Co. LLC
Tin powder: tin powder having a diameter of 45 µm or less, available from Sigma-Aldrich Co. LLC
Titanium powder: titanium powder having a diameter of 45 µm or less, available from Sigma-Aldrich Co. LLC
Copper powder: copper powder having a diameter of 45 µm or less, available from Sigma-Aldrich Co. LLC Carbon black: DENKA BLACK Li-400, available from Denka Co., Ltd.

Multilayer carbon nanotube: multilayer carbon nanotube having a diameter of 110 to 170 nm and a length of 5 to 9 µm, available from Sigma-Aldrich Co. LLC

### [Crosslinking Agent]

A crosslinking agent, which was incorporated into the bio-electrode compositions of Examples and Comparative Examples, is shown below.

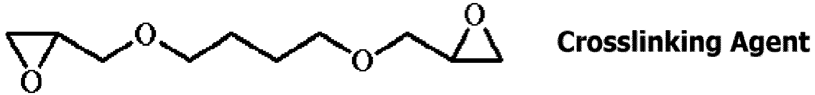

### [Organic Solvent (E)]

An organic solvent (E), which was incorporated into the bio-electrode compositions of Examples and Comparative Examples, is shown below.
EDE: diethylene glycol diethyl ether
BE: diethylene glycol butyl ether
Isopar-G (Exxon Mobil Corporation): isoparaffin

### [Additives]

A platinum catalyst and a reaction inhibitor, which were incorporated into the bio-electrode compositions of Examples and Comparative Examples as additives, are shown below.
Platinum catalyst: CAT-PL-56, available from Shin-Etsu Chemical Co., Ltd.
Reaction inhibitor: 1-ethynylcyclohexanol, available from Tokyo Chemical Industry Co., Ltd.

### [Ionic Material]

Ionic polymer 1 used in Comparative Examples was synthesized as follows. 30 mass% solution of corresponding monomers in cyclopentanone was introduced into a reaction container and mixed. The inside of the reaction container was cooled to -70°C under a nitrogen atmosphere, followed by vacuum degassing and nitrogen blowing, which were repeated three times. After raising the temperature to room temperature, azobisisobutyronitrile (AIBN) was added thereto as a polymerization initiator in an amount of 0.01 moles per 1 mole of the whole monomers. The resultant was heated to 60°C, and then allowed to react for 15 hours. After drying the solvent, the composition of the obtained polymer was confirmed by ¹H-NMR. Further, the molecular weight (Mw) and the dispersity (Mw/Mn) of the obtained polymer were determined by gel permeation chromatography (GPC) using tetrahydrofuran (THF) as a solvent. The measurement temperature at this time was 40°C. The ionic polymer 1 synthesized as above is shown below.
Ionic polymer 1
Mw = 44,400
Mw/Mn = 1.94

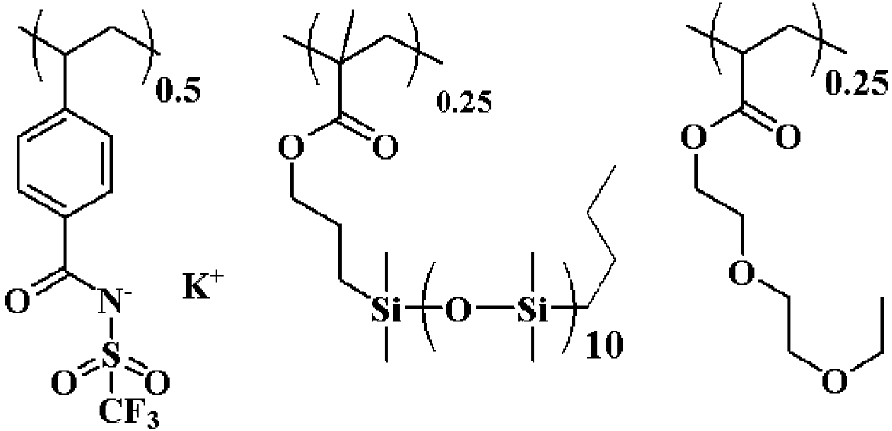

The repeating number in each formula shows the average value.

### Examples 1 to 22 and Comparative Examples 1 to 7

Bio-electrode compositions (bio-electrode compositions 1 to 22 and comparative bio-electrode compositions 1 to 7) were prepared by blending a deep eutectic liquid, a binder resin, conductive particles, an organic solvent, additives (such as a platinum catalyst, a reaction inhibitor, and a crosslinking agent), and an ionic material in accordance with the formulations shown in Tables 2 to 4.

**[Table 2]**

| Bio-electrode Composition | Deep Eutectic Liquid (part by mass) | Binder Resin (part by mass) | Conductive Particles (part by mass) | Organic Solvent (part by mass) | Additives (part by mass) |
|---|---|---|---|---|---|
| Bio-electrode Composition 1 | Deep Eutectic Liquid 1 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 2 | Deep Eutectic Liquid 2 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 3 | Deep Eutectic Liquid 3 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 4 | Deep Eutectic Liquid 4 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 5 | Deep Eutectic Liquid 5 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 6 | Deep Eutectic Liquid 6 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 7 | Deep Eutectic Liquid 7 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 8 | Deep Eutectic Liquid 8 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 9 | Deep Eutectic Liquid 9 (15) | Siloxane Compound 1 (40) | Carbon Black (10) | Isoper-G (20) | CAT-PL-56 (2.9) |
| | | Siloxane Compound 2 (100) | | | 1-Ethynylcyclohexanol (1.4) |
| | | Siloxane Compound 4 (1) | | | |
| Bio-electrode Composition 10 | Deep Eutectic Liquid 1 (15) | Siloxane Compound 1 (40) | Carbon Black (10) | Isoper-G (20) | CAT-PL-56 (3) |
| | | Siloxane Compound 2 (100) | | | |
| | | Siloxane Compound 4 (1) | Silver Flake (8) | | |
| Bio-electrode Composition 11 | Deep Eutectic Liquid 1 (15) | Siloxane Compound 1 (40) | Carbon Black (10) | Isoper-G (20) | CAT-PL-56 (3) |
| | | Siloxane Compound 2 (100) | | | |
| | | Siloxane Compound 4 (1) | Gold Powder (5) | | |
| Bio-electrode Composition 12 | Deep Eutectic Liquid 1 (15) | Siloxane Compound 1 (40) | Carbon Black (10) | Isoper-G (20) | CAT-PL-56 (3) |
| | | Siloxane Compound 2 (100) | | | |
| | | Siloxane Compound 4 (1) | Tin Powder (5) | | |
| Bio-electrode Composition 13 | Deep Eutectic Liquid 1 (15) | Siloxane Compound 1 (40) | Carbon Black (10) | Isoper-G (20) | CAT-PL-56 (3) |
| | | Siloxane Compound 2 (100) | | | |
| | | Siloxane Compound 4 (1) | Titanium Powder (5) | | |
| Bio-electrode Composition 14 | Deep Eutectic Liquid 1 (15) | Siloxane Compound 1 (40) | Carbon Black (10) | Isoper-G (20) | CAT-PL-56 (3) |
| | | Siloxane Compound 2 (100) | | | |
| | | Siloxane Compound 4 (1) | Copper Powder (5) | | |
| Bio-electrode Composition 15 | Deep Eutectic Liquid 1 (15) | Siloxane Compound 1 (40) | Carbon Black (10) | Isoper-G (20) | CAT-PL-56 (5) |
| | | Siloxane Compound 2 (100) | Multilayer Carbon Nanotube (3) | | |
| | | Siloxane Compound 4 (1) | | | |
| Bio-electrode Composition 16 | Deep Eutectic Liquid 1 (15) | Siloxane Compound 3 (126) | Carbon Black (15) | Isoper-G (20) | CAT-PL-56 (5) |
| | | Siloxane Compound 4 (3) | | | |

**[Table 3]**

| Bio-electrode Composition | Deep Eutectic Liquid (part by mass) | Binder Resin (part by mass) | Conductive Particles (part by mass) | Organic Solvent (part by mass) | Additives (part by mass) |
|---|---|---|---|---|---|
| Bio-electrode Composition 17 | Deep Eutectic Liquid 1 (30) | Acrylic Polymer 1 (100) | Carbon Black (14) | BE (200) | Crosslinking Agent (2) |
| Bio-electrode Composition 18 | Deep Eutectic Liquid 1 (30) | Acrylic Polymer 1 (25) | Carbon Black (14) | BE (50) | Crosslinking Agent (0.5) |
| | | Urethane Resin 2 (75) | | EDE (25) | |
| Bio-electrode Composition 19 | Deep Eutectic Liquid 1 (30) | Acrylic Polymer 1 (25) | Carbon Black (14) | BE (50) | Crosslinking Agent (0.5) |
| | | Urethane Resin 3 (75) | | EDE (25) | |
| Bio-electrode Composition 20 | Deep Eutectic Liquid 1 (30) | Urethane Resin 1 (100) | Carbon Black (14) | EDE (33) | - |
| Bio-electrode Composition 21 | Deep Eutectic Liquid 1 (30) | Urethane Resin 2 (100) | Carbon Black (14) | EDE (33) | - |
| Bio-electrode Composition 22 | Deep Eutectic Liquid 1 (30) | Urethane Resin 3 (100) | Carbon Black (14) | EDE (33) | - |

**[Table 4]**

| Comparative Bio-electrode Compositions | Ionic Material (part by mass) | Binder Resin (part by mass) | Conductive Particles (part by mass) | Organic Solvent (part by mass) | Additives (part by mass) |
|---|---|---|---|---|---|
| Comparative Bio-electrode Composition 1 | Ionic Polymer 1 (15) | Siloxane Compound 1 (40) | Carbon Black (10) | Isoper-G (35) | CAT-PL-56 (5) |
| | | Siloxane Compound 2 (100) | | Cyclopentanone (60) | |
| | | Siloxane Compound 4 (1) | | | |
| Comparative Bio-electrode Composition 2 | Ionic Polymer 1 (5) | Siloxane Compound 1 (40) | Carbon Black (10) | Isoper-G (35) | CAT-PL-56 (5) |
| | | Siloxane Compound 2 (100) | | | |
| | | | | Cyclopentanone (60) | |
| | | | | | KF-6100 (5) |
| | | Siloxane Compound 4 (1) | | | |
| Comparative Bio-electrode Composition 3 | - | Siloxane Compound 1 (40) | Carbon Black (10) | Isoper-G (35) | CAT-PL-56 (5) |
| | | Siloxane Compound 2 (100) | | | |
| | | | | Cyclopentanone (60) | |
| | | | | | KF-6100 (15) |
| | | Siloxane Compound 4 (1) | | | |
| Comparative Bio-electrode Composition 4 | Ionic Polymer 1 (5) | Siloxane Compound 1 (40) | Carbon Black (10) | Isoper-G (35) | CAT-PL-56 (5) |
| | | Siloxane Compound 2 (100) | | | |
| | | | | Cyclopentanone (60) | Polyglycerin #310 (5) |
| | | Siloxane Compound 4 (1) | | | |
| Comparative Bio-electrode Composition 5 | - | Siloxane Compound 1 (40) | Carbon Black (10) | Isoper-G (35) | CAT-PL-56 (5) |
| | | Siloxane Compound 2 (100) | | | |
| | | | | Cyclopentanone (60) | Polyglycerin #310 (15) |
| | | Siloxane Compound 4 (1) | | | |
| Comparative Bio-electrode Composition 6 | Betaine Anhydrous (10) | Acrylic Polymer 1 (80) | Carbon Black (15) | BE (60) | - |
| | | | | Water (50) | |
| Comparative Bio-electrode Composition 7 | Choline Chloride (10) | Acrylic Polymer 1 (80) | Carbon Black (15) | BE (60) | - |
| | | | | Water (50) | |

### (Preparation of Samples for Biological Signal Evaluation)

A thermoplastic urethane (TPU) film ST-604 (available from Bemis Associates Inc.) was coated with a conductive paste DOTITE FA-333 (available from Fujikura Kasei Co., Ltd.) by screen printing. The coating film was baked in an oven at 120°C for 10 minutes to print a keyhole-shaped conductive pattern including a circular portion with a diameter of 2 cm and a rectangular portion. Then, each of the bio-electrode compositions shown in Tables 2 to 4 was applied onto the circular portion of the printed conductive pattern by screen printing. After the coating film was air-dried at room temperature for 10 minutes, the coating film was cured by being baked in an oven at 125°C for 10 minutes to evaporate the solvent. By the curing, a living body contact layer, which is a cured product of each bio-electrode composition, was obtained (Examples 1 to 22, Comparative Examples 1 to 7).

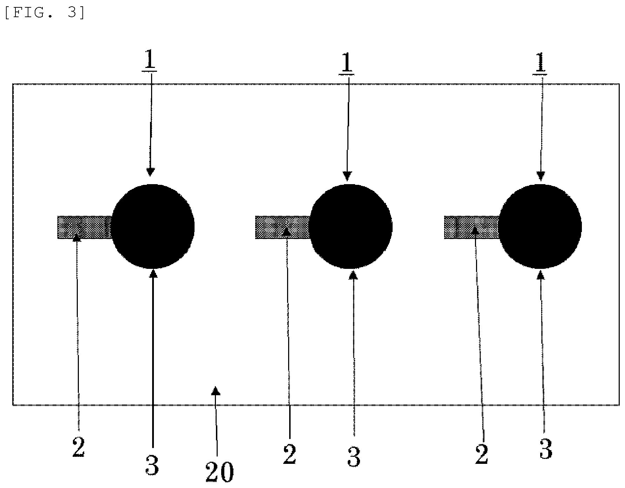

FIG. 3 is a schematic-view of printed bio-electrodes prepared in the Examples. As shown in FIG. 3, a plurality of bio-electrodes 1 were fabricated on a thermoplastic urethane film 20. Each bio-electrode 1 included a keyhole-shaped conductive pattern 2 as a conductive base material and a living body contact layer 3 formed over the circular portion of the conductive pattern 2.

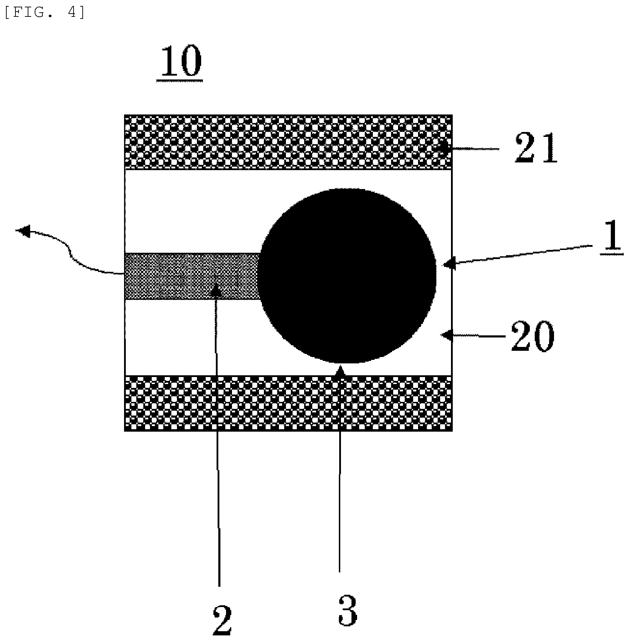

Next, as shown in FIG. 4, the thermoplastic urethane film 20 having the bio-electrode 1 printed thereon was cut out and a double-sided tape 21 was bonded thereto. In this manner, three bio-electrode samples 10 (samples for biological signal evaluation) were prepared for a single bio-electrode composition.

### (Thickness Measurement of Living Body Contact Layer)

The thickness of the living body contact layer in each bio-electrode sample prepared as described above was measured with a micrometer. Table 5 shows the results.

### (Measurement of Biological Signals)

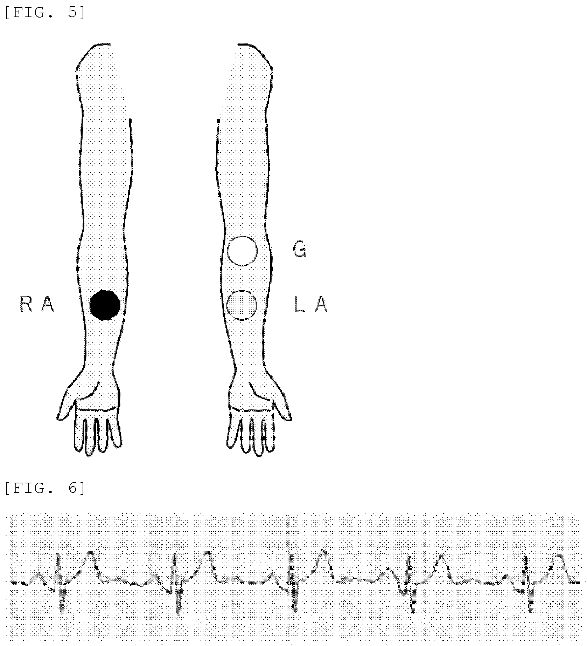

A conductive wiring pattern formed from a conductive paste of a bio-electrode was connected via conductive wires to the NeXus-10 MARK II (a multi-sensor physiological measurement system), which is available from MindMedia, Netherlands. The positive electrode of the electrocardiograph was attached to the position LA on the left forearm, the negative electrode to the position RA on the right forearm, and the ground to the position G, as shown in FIG. 5. The electrocardiogram measurement was started immediately after the attachment, and the initial potential of the electrocardiographic waveform including P, Q, R, S, and T waves, as shown in FIG. 6, as well as the baseline variation of the electrocardiographic waveform, were evaluated. Table 5 shows the results. In addition, immediately before the attachment, the electrode surface or the skin may be wiped with a gauze impregnated with a solution containing 70% ethanol and 30% water.

### [Criteria for Assessing Initial Potential of ECG Signal]

AA: The potential is -50,000 µV or more and less than +50,000 µV.
A: The potential is -100,000 µV or more and less than - 50,000 µV, or +50,000 µV or more and less than +100,000 µV.
B: The potential is less than -100,000 µV or +100,000 µV or more.
C: The initial electrocardiogram is not measurable.

### Criteria for Assessing Baseline Potential Variation of ECG Signal

A: The potential variation from the initial potential is less than 20,000 µV during the 10-minute ECG measurement.
B: The potential variation from the initial potential is 20,000 µV or more and less than 50,000 µV during the 10-minute ECG measurement.
C: The potential variation from the initial potential is 50,000 µV or more during the 10-minute ECG measurement.

**[Table 5]**

| Examples | Bio-electrode Compositions | Thickness of Living Body Contact Layer (µm) | Initial Potential of ECG Signal | Baseline Potential Variation of ECG Signal |
|---|---|---|---|---|
| Example 1 | Bio-electrode Composition 1 | 100 | AA | A |
| Example 2 | Bio-electrode Composition 2 | 101 | AA | A |
| Example 3 | Bio-electrode Composition 3 | 98 | A | A |
| Example 4 | Bio-electrode Composition 4 | 98 | AA | A |
| Example 5 | Bio-electrode Composition 5 | 101 | A | A |
| Example 6 | Bio-electrode Composition 6 | 100 | A | A |
| Example 7 | Bio-electrode Composition 7 | 99 | A | A |
| Example 8 | Bio-electrode Composition 8 | 100 | A | A |
| Example 9 | Bio-electrode Composition 9 | 100 | AA | A |
| Example 10 | Bio-electrode Composition 10 | 99 | AA | A |
| Example 11 | Bio-electrode Composition 11 | 103 | AA | A |
| Example 12 | Bio-electrode Composition 12 | 102 | AA | A |
| Example 13 | Bio-electrode Composition 13 | 100 | AA | A |
| Example 14 | Bio-electrode Composition 14 | 97 | AA | A |
| Example 15 | Bio-electrode Composition 15 | 99 | AA | A |
| Example 16 | Bio-electrode Composition 16 | 104 | AA | A |
| Example 17 | Bio-electrode Composition 17 | 98 | A | A |
| Example 18 | Bio-electrode Composition 18 | 101 | A | A |
| Example 19 | Bio-electrode Composition 19 | 101 | A | A |
| Example 20 | Bio-electrode Composition 20 | 102 | A | A |
| Example 21 | Bio-electrode Composition 21 | 99 | A | A |
| Example 22 | Bio-electrode Composition 22 | 99 | A | A |
| Comparative Example 1 | Comparative Bio-electrode Composition 1 | 103 | C | - |
| Comparative Example 2 | Comparative Bio-electrode Composition 2 | 103 | A | C |
| Comparative Example 3 | Comparative Bio-electrode Composition 3 | 102 | A | C |
| Comparative Example 4 | Comparative Bio-electrode Composition 4 | 100 | B | C |
| Comparative Example 5 | Comparative Bio-electrode Composition 5 | 101 | B | C |
| Comparative Example 6 | Comparative Bio-electrode Composition 6 | 102 | C | - |
| Comparative Example 7 | Comparative Bio-electrode Composition 7 | 105 | C | - |

As shown in Table 5, in Examples 1 to 22 in which a living body contact layer was formed from a cured product of one of the bio-electrode compositions 1 to 22 each containing the deep eutectic liquid of the present invention having the specific structure described above, the binder resin (A), and the conductive particles (B), biological signals (ECG signal) were obtained immediately after the attachment to the body.

On the other hand, the comparative bio-electrode composition 1 of Comparative Example 1 contained an ionic polymer compound, the binder resin (A), and the conductive particles (B), but did not contain a deep eutectic liquid. As a result, the comparative bio-electrode composition 1 of Comparative Example 1 failed to obtain biological signals from the beginning of the measurement. This is presumably because the ionic polymer, which is in a solid state, exhibited inferior ionic conductivity compared to the deep eutectic liquid.

Furthermore, the comparative bio-electrode compositions 2 and 4 of Comparative Examples 2 and 4 contained an ionic polymer compound, the binder resin (A), the conductive particles (B), and the hydrogen bond donor compound used in the Examples, but did not contain a hydrogen bond acceptor compound and thus did not contain a deep eutectic liquid. As a result, although biological signals were obtained from the beginning of the measurement, the baseline potential variation of the ECG signal was large. The reasons are assumed as follows. The incorporation of the hydrogen bond donor compound provided desirable adhesion to the living body, enabling acquisition of biological signals from the beginning; however, due to the poor ionic conductivity of the electrode, a polarization voltage was generated between the living body and the electrode, leading to large potential variations.

Furthermore, although the comparative bio-electrode compositions 3 and 5 of Comparative Examples 3 and 5 did not contain an ionic polymer compound, the results had no significant difference depending on the presence or absence of the ionic polymer compound. This is presumably due to insufficient ionic conductivity of the ionic polymer compound, which resulted in the generation of a polarization voltage between the living body and the electrode, leading to large potential variations.

The comparative bio-electrode compositions 6 and 7 of Comparative Examples 6 and 7 contained choline chloride and betaine anhydrous as ionic materials; however, since these are solids, water was used to disperse them. However, it is considered that, when these compositions are applied and made into a film, the materials precipitated as solids, resulting in the absence of ionic conductivity and the inability to obtain biological signals.

From the above, it has been clarified that the deep eutectic liquid of the present invention, the bio-electrode composition containing the deep eutectic liquid, the bio-electrode formed using the composition, and the method for producing the bio-electrode enable prompt and stable acquisition of biological signals. That is, the deep eutectic liquid of the present invention, the bio-electrode composition containing the deep eutectic liquid, the bio-electrode formed using the composition, and the method for producing the bio-electrode make it possible to provide a bio-electrode composition and a bio-electrode that are capable of prompt and stable acquisition of biological signals when attached to a living body.

The present description includes the following embodiments.
[1]: A deep eutectic liquid, which is a mixture of a hydrogen bond donor compound and a hydrogen bond acceptor compound, wherein the hydrogen bond donor compound is a compound represented by the following general formula (1) having a structure in which 2 to 100 monomers having a hydroxy group are bonded, the hydrogen bond acceptor compound is a compound containing a monomer having a quaternary ammonium cation represented by the following general formulae (2) to (6) or a quaternary phosphonium cation represented by the following general formula (7), and the deep eutectic liquid is present in a liquid state at 25°C, 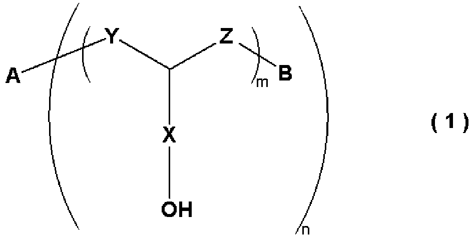 wherein X is a single bond, or a linear, branched, or cyclic divalent hydrocarbon group having 1 to 30 carbon atoms, which may be substituted with a heteroatom or intervened by a heteroatom; Y and Z each represent a linear, branched or cyclic divalent hydrocarbon group having 1 to 5 carbon atoms, which may be substituted with a heteroatom or intervened by a heteroatom; A and B each represent a hydrogen atom, a hydroxy group, an amino group, a halogen atom, or an alkyl group or an alkyl group with a terminal substituted with a siloxane that may be substituted with a heteroatom or intervened by a heteroatom; Y and Z may be identical to or different from each other; A and B may be identical to or different from each other; "m" is an integer of 1 to 100 and represents a repetition of chemical structural units, and "n" is an integer of 1 to 4 and represents a repetition of chemical structural units, provided that 2 ≤ m×n ≤ 100 is satisfied, 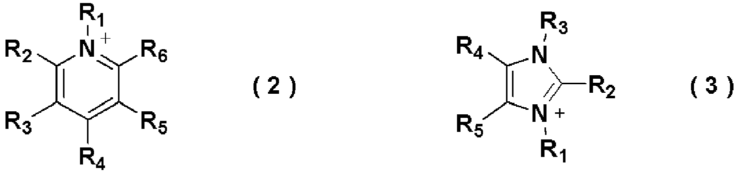 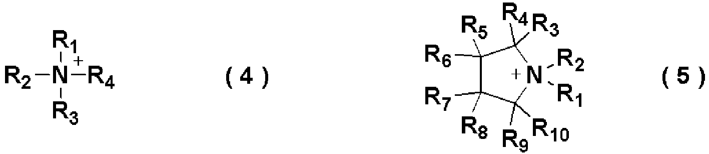 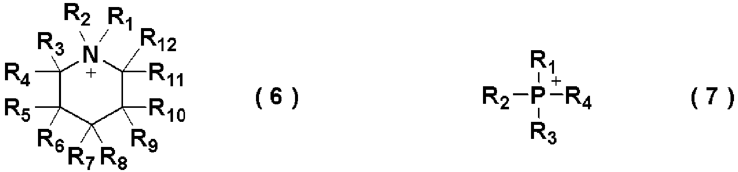 wherein R₁ to R₁₂ each represent a linear, branched, or cyclic monovalent hydrocarbon group having 1 to 30 carbon atoms that may be substituted with a heteroatom or intervened by a heteroatom and may form a zwitterion having an anionic portion, or a hydrogen atom, a hydroxy group, an amino group, a nitro group, or a halogen atom; R₁ to R₁₂ may be identical to or different from one another.
[2]: The deep eutectic liquid according to the above [1], wherein each monomer having a hydroxy group is glycerin.
[3]: The deep eutectic liquid according to the above [2], wherein the hydrogen bond donor compound is a polyglycerin-modified silicone represented by the following general formula (8) or (9), 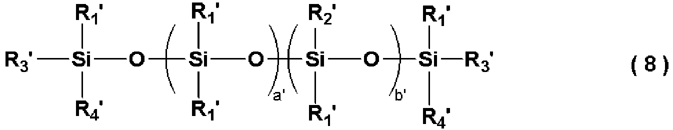 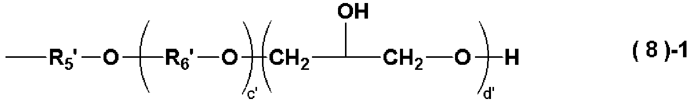 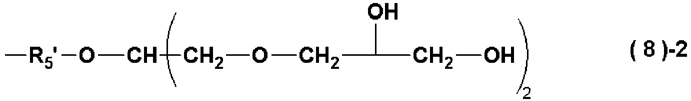 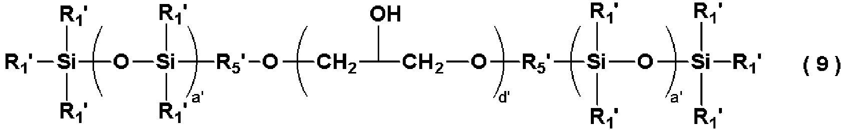 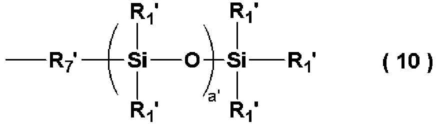 wherein R₁' is identical to or different from one another, and independently represents a hydrogen atom, a phenyl group, a linear or branched alkyl group having 1 to 50 carbon atoms, or a silicone chain represented by the general formula (10), and optionally contains an ether group; R₂' represents a group having a polyglycerin structure represented by the general formula (8)-1 or (8)-2; R₃' is identical to or different from one another, and independently represents the R₁' or the R₂'; R₄' is identical to or different from one another, and independently represents the R₁', the R₂', or an oxygen atom; when R₄' is an oxygen atom, two R₄' moieties may bond to each other to form an ether group and may form a ring together with silicon atoms to which they are bonded; "a'" is identical to or different from one another and represents 0 to 100, "b'" is 0 to 100, and a'+b' is 0 to 200, provided that, when "b'" is 0, at least one of R₃' is the R₂'; R₅' represents an alkylene group having 2 to 10 carbon atoms or an aralkylene group having 7 to 10 carbon atoms; R₆' and R₇' each represent an alkylene group having 2 to 6 carbon atoms; R₇' may be an ether group; "c'" is 0 to 20; and "d'" is 2 to 20.
[4]: A bio-electrode composition, comprising the deep eutectic liquid according to any one of the above [1] to [3].
[5]: The bio-electrode composition according to the above [4], wherein the bio-electrode composition comprises a binder (A).
[6]: The bio-electrode composition according to the above [5], wherein the binder (A) is one or more resins selected from the group consisting of a silicone resin, a polyurethane resin, and a polyacrylic resin.
[7]: The bio-electrode composition according to any one of the above [4] to [6], wherein the bio-electrode composition comprises a conductive particle (B).
[8]: The bio-electrode composition according to the above [7], wherein the conductive particle (B) comprises one or more selected from the group consisting of carbon powder, gold, silver, silver chloride, platinum, aluminum, magnesium, tin, tungsten, iron, copper, nickel, stainless steel, chromium, titanium, molybdenum, ruthenium, and indium.
[9]: The bio-electrode composition according to the above [8], wherein the carbon powder is one or both of carbon black and carbon nanotube.
[10]: The bio-electrode composition according to any one of the above [4] to [9], wherein the bio-electrode composition further comprises glycerin.
[11]: A bio-electrode comprising a conductive base material and a living body contact layer formed on the conductive base material, wherein the living body contact layer comprises a cured product of the bio-electrode composition according to any one of the above [4] to [10].
[12]: The bio-electrode according to the above [11], wherein the conductive base material comprises one or more selected from the group consisting of gold, silver, silver chloride, platinum, aluminum, magnesium, tin, tungsten, iron, copper, nickel, stainless steel, chromium, titanium, and carbon.
[13]: A method for producing a bio-electrode having a conductive base material and a living body contact layer formed on the conductive base material,
   the method comprising:
   applying the bio-electrode composition according to any one of the above [4] to [10] onto the conductive base material; and
   curing the bio-electrode composition to form the living body contact layer.
[14]: The method for producing a bio-electrode according to the above [13], wherein the conductive base material comprises one or more selected from the group consisting of gold, silver, silver chloride, platinum, aluminum, magnesium, tin, tungsten, iron, copper, nickel, stainless steel, chromium, titanium, and carbon.
[15]: A method for producing a bio-electrode having a conductive base material and a living body contact layer formed on the conductive base material,
   the method comprising:
   applying the bio-electrode composition according to any one of the above [4] to [10] onto a release substrate, followed by curing of the bio-electrode composition, and patterning of the cured product; and
   transferring the patterned product onto the conductive base material to form the living body contact layer.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A deep eutectic liquid, which is a mixture of a hydrogen bond donor compound and a hydrogen bond acceptor compound, wherein the hydrogen bond donor compound is a compound represented by the following general formula (1) having a structure in which 2 to 100 monomers having a hydroxy group are bonded, the hydrogen bond acceptor compound is a compound containing a monomer having a quaternary ammonium cation represented by the following general formulae (2) to (6) or a quaternary phosphonium cation represented by the following general formula (7), and the deep eutectic liquid is present in a liquid state at 25°C, 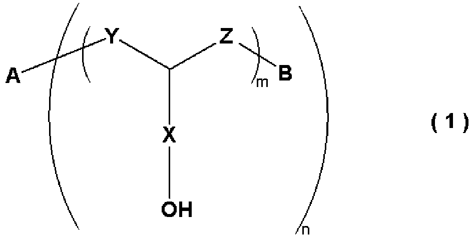 wherein X is a single bond, or a linear, branched, or cyclic divalent hydrocarbon group having 1 to 30 carbon atoms, which may be substituted with a heteroatom or intervened by a heteroatom; Y and Z each represent a linear, branched or cyclic divalent hydrocarbon group having 1 to 5 carbon atoms, which may be substituted with a heteroatom or intervened by a heteroatom; A and B each represent a hydrogen atom, a hydroxy group, an amino group, a halogen atom, or an alkyl group or an alkyl group with a terminal substituted with a siloxane that may be substituted with a heteroatom or intervened by a heteroatom; Y and Z may be identical to or different from each other; A and B may be identical to or different from each other; "m" is an integer of 1 to 100 and represents a repetition of chemical structural units, and "n" is an integer of 1 to 4 and represents a repetition of chemical structural units, provided that 2 ≤ m×n ≤ 100 is satisfied, 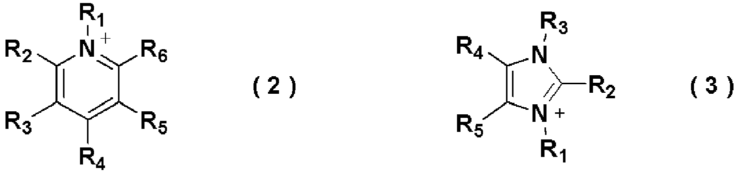 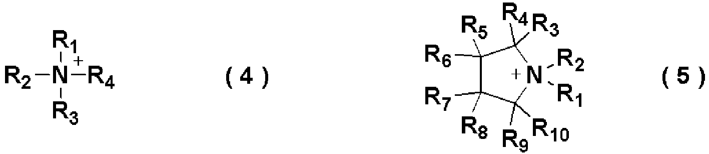 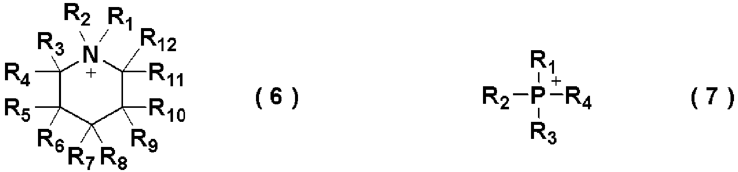 wherein R₁ to R₁₂ each represent a linear, branched, or cyclic monovalent hydrocarbon group having 1 to 30 carbon atoms that may be substituted with a heteroatom or intervened by a heteroatom and may form a zwitterion having an anionic portion, or a hydrogen atom, a hydroxy group, an amino group, a nitro group, or a halogen atom; R₁ to R₁₂ may be identical to or different from one another.

2. The deep eutectic liquid according to claim 1, wherein each monomer having a hydroxy group is glycerin.

3. The deep eutectic liquid according to claim 2, wherein the hydrogen bond donor compound is a polyglycerin-modified silicone represented by the following general formula (8) or (9), 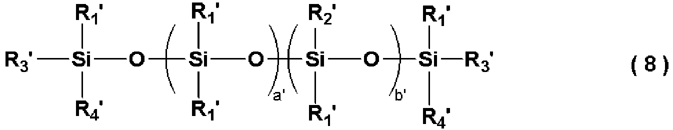 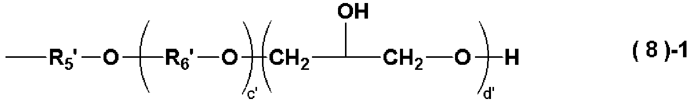 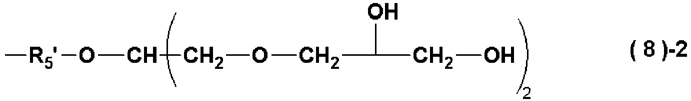 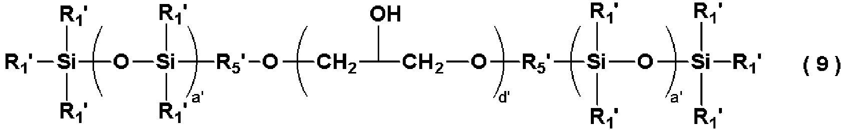 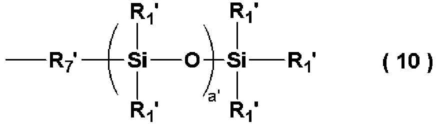 wherein R₁' is identical to or different from one another, and independently represents a hydrogen atom, a phenyl group, a linear or branched alkyl group having 1 to 50 carbon atoms, or a silicone chain represented by the general formula (10), and optionally contains an ether group; R₂' represents a group having a polyglycerin structure represented by the general formula (8)-1 or (8)-2; R₃' is identical to or different from one another, and independently represents the R₁' or the R₂'; R₄' is identical to or different from one another, and independently represents the R₁', the R₂', or an oxygen atom; when R₄' is an oxygen atom, two R₄' moieties may bond to each other to form an ether group and may form a ring together with silicon atoms to which they are bonded; "a'" is identical to or different from one another and represents 0 to 100, "b'" is 0 to 100, and a'+b' is 0 to 200, provided that, when "b'" is 0, at least one of R₃' is the R₂'; R₅' represents an alkylene group having 2 to 10 carbon atoms or an aralkylene group having 7 to 10 carbon atoms; R₆' and R₇' each represent an alkylene group having 2 to 6 carbon atoms; R₇' may be an ether group; "c'" is 0 to 20; and "d'" is 2 to 20.

4. A bio-electrode composition, comprising the deep eutectic liquid according to any one of claims 1 to 3.

5. The bio-electrode composition according to claim 4, wherein the bio-electrode composition comprises a binder (A).

6. The bio-electrode composition according to claim 5, wherein the binder (A) is one or more resins selected from the group consisting of a silicone resin, a polyurethane resin, and a polyacrylic resin.

7. The bio-electrode composition according to any one of claims 4 to 6, wherein the bio-electrode composition comprises a conductive particle (B).

8. The bio-electrode composition according to claim 7, wherein the conductive particle (B) comprises one or more selected from the group consisting of carbon powder, gold, silver, silver chloride, platinum, aluminum, magnesium, tin, tungsten, iron, copper, nickel, stainless steel, chromium, titanium, molybdenum, ruthenium, and indium.

9. The bio-electrode composition according to claim 8, wherein the carbon powder is one or both of carbon black and carbon nanotube.

10. The bio-electrode composition according to any one of claims 4 to 9, wherein the bio-electrode composition further comprises glycerin.

11. A bio-electrode comprising a conductive base material and a living body contact layer formed on the conductive base material, wherein the living body contact layer comprises a cured product of the bio-electrode composition according to any one of claims 4 to 10.

12. The bio-electrode according to claim 11, wherein the conductive base material comprises one or more selected from the group consisting of gold, silver, silver chloride, platinum, aluminum, magnesium, tin, tungsten, iron, copper, nickel, stainless steel, chromium, titanium, and carbon.

13. A method for producing a bio-electrode having a conductive base material and a living body contact layer formed on the conductive base material,
the method comprising:
applying the bio-electrode composition according to any one of claims 4 to 10 onto the conductive base material; and
curing the bio-electrode composition to form the living body contact layer.

14. The method for producing a bio-electrode according to claim 13, wherein the conductive base material comprises one or more selected from the group consisting of gold, silver, silver chloride, platinum, aluminum, magnesium, tin, tungsten, iron, copper, nickel, stainless steel, chromium, titanium, and carbon.

15. A method for producing a bio-electrode having a conductive base material and a living body contact layer formed on the conductive base material,
the method comprising:
applying the bio-electrode composition according to any one of claims 4 to 10 onto a release substrate, followed by curing of the bio-electrode composition, and patterning of the cured product; and
transferring the patterned product onto the conductive base material to form the living body contact layer.
